# EUROPÄISCHE PATENTSCHRIFT

(11) **EP 3 136 991 B1**
(45) Veröffentlichungstag und Bekanntmachung des Hinweises auf die Patenterteilung: **07.07.2021**
(21) Anmeldenummer: 15721153.3
(22) Anmeldetag: 24.04.2015
(51) Int. Cl.: A61B 17/128, A61B 17/29

(54) **MAULTEIL MIT SCHICHTAUFBAU FÜR EIN CHIRURGISCHES INSTRUMENT**
JAW PIECE WITH A LAYERED CONSTRUCTION, FOR A SURGICAL INSTRUMENT
MÂCHOIRE À STRUCTURE STRATIFIÉE POUR INSTRUMENT CHIRURGICAL

(30) Priorität: 28.04.2014 DE 102014207971
(43) Veröffentlichungstag der Anmeldung: 08.03.2017
(73) Patentinhaber: Aesculap AG, 78532 Tuttlingen (DE)
(72) Erfinder: SCHOLTEN, Thomas, 78532 Tuttlingen (DE); WANKE, Gunnar, CH-8280 Kreuzlingen (CH)
(74) Vertreter: Winter, Brandl - Partnerschaft mbB
(86) Internationale Anmeldenummer: PCT/EP2015/058942
(87) Internationale Veröffentlichungsnummer: WO 2015/165819

(56) Entgegenhaltungen:
- EP-A2- 2 481 360
- WO-A2-2006/042141
- US-B1- 6 228 097
- None

## Beschreibung

Die vorliegende Erfindung betrifft ein Maulteil für ein chirurgisches Rohrschaft-Instrument und insbesondere ein Maulteil, bei dem Kulissenelemente am hinteren Ende der Branchen ausgebildet sind. Ein chirurgisches Rohrschaft-Instrument ist beispielsweise ein endoskopisches Rohrschaft-Instrument zum Applizieren von chirurgischen Clips.

### Stand der Technik

Im Stand der Technik sind einige Maulteile für chirurgische Rohrschaft-Instrumente bekannt. So ist in der europäischen Patentanmeldung EP 1 712 187 A2 beispielsweise ein Maulteil gezeigt, bei dem die beiden Branchen elastisch federnd über eine gemeinsame Basis verbunden sind. im Bereich ihrer distalen Enden, welche dazu vorgesehen sind, den chirurgischen Clip zu halten und zusammen zu drücken und den Clip dadurch zu applizieren, weisen die beiden Branchen auf ihren Außenseiten je eine Gleitfläche auf. Um das Maulteil zu schließen und so den Clip zu applizieren, wird das Maulteil gegenüber dem Schaft, in dem es angeordnet ist, nach proximal verschoben (das Maulteil wird also teilweise zu dem Griffstück hin in den Schaft hinein gezogen bzw. der Schaft wird über das Maulteil geschoben) und der distale Rand des Schafts gleitet an den Gleitflächen entlang. Durch die Schrägstellung der Gleitflächen gegenüber der Achse des Schafts werden die distalen Enden der Branchen nach innen gedrängt, während die proximalen Enden der Branchen durch die Basis gehalten werden. Auf diese Weise führen die Branchen jeweils eine Drehbewegung um den Punkt herum aus, an dem die Branchen mit der Basis verbunden sind. Ein Öffnungsvorgang des Maulteils erfolgt ohne Führung und wird ausschließlich durch die Elastizität der Branchen gewährleistet, die in ihre Ausgangsposition zurück drängen, wenn das Maulteil während des Öffnungsvorgangs aus dem Schaft heraus geschoben wird.

Ein vergleichbares Maulteil ist auch in der internationalen Patentanmeldung WO 2008/127 968 gezeigt, auch wenn sich das dort gezeigte Instrument insgesamt stark von dem vorstehend beschriebenen Instrument unterscheidet.

Noch deutlicher wird die Drehbewegung der Branchen beim Öffnen und Schließen des Maulteils aus der US Patentanmeldung US 2005/0171560 A1. Dort sind die distalen Bereiche beider Branchen an der Basis gelenkig angebracht und drehen sich um den Befestigungspunkt. Auch bei dieser Konstruktion wird der Clip appliziert, indem der distale Rand des Schafts an den Gleitflächen, welche an den Außenseiten der Branchen vorgesehen sind, entlang gleitet und so die Branchen nach innen drängt.

Das Problem dieser Art von Maulteilen ist, dass sie eine immer gleiche Schließgeometrie aufweisen, genauer gesagt, dass sich immer zuerst die distalen Enden der Branchen berühren bzw. aneinander vorbei gleiten und sich der Kontakt bzw. das aneinander vorbei Gleiten der weiter proximal liegenden Bereiche der Branchen dem anschließt. Bei Clipapplikatoren bedeutet dies, dass der Clip immer vom distalen Ende her geschlossen wird. Für andere chirurgische Instrumente wie beispielsweise eine endoskopische Schere ist dieser Aufbau eines Maulteils aus diesem Grund nicht brauchbar.

Ein weiteres Problem dieser Art von Maulteilen ist, dass das Öffnen des Maulteils alleine durch die Elastizität der Branchen realisiert wird. Die Öffnungsbewegung des Maulteils erfolgt ohne Führung. Sollte ein Gewebestück oder ein anderes Teil zwischen den vorderen Rand des Schaftes und eine Branche des Maulteils gelangen, könnte dies den Öffnungsvorgang des Maulteils behindern. Dann müsste das Instrument erst aus dem Körper des Patienten entnommen werden, um von dem Gewebestück befreit zu werden, um anschließend wieder in den Patienten eingeführt zu werden. Dies führt zu Verzögerungen und Störungen im Operationsablauf.

Aus dem Stand der Technik sind WO 2006/042141 A1, US 6 228 097 B1 und EP 2 481 360 A2 bekannt.

### Aufgabe der Erfindung

Die Aufgabe der vorliegenden Erfindung ist es, ein Maulteil für ein chirurgisches Rohrschaft-Instrument bereit zu stellen, welches es ermöglicht, Clips aus einem Magazin von proximal in das Maulteil einzuführen, ein Verfahren zum Zusammenbau eines Maulteils für ein chirurgisches Rohrschaft-Instrument sowie ein Verfahren zum Zusammenbau eines chirurgischen Rohrschaft-Instruments. Die Aufgabe der vorliegenden Erfindung wird durch ein Maulteil nach Anspruch 1 oder 14 und Verfahren nach Ansprüchen 17 und 18 gelöst. Vorteilhafte Weiterbildungen und Ausgestaltungen der Maulteile sind Gegenstand der abhängigen Ansprüche. Begriffsdefinitionen

Der Begriff des chirurgischen Rohrschaft-Instruments umfasst in dieser Anmeldung zum einen endoskopische Instrumente wie beispielsweise endoskopische Clipapplikatoren oder Nadelhalter. Zum anderen umfasst dieser Begriff aber auch chirurgische Instrumente für eine offene Operation, bei denen der Funktionsabschnitt bzw. der Wirkabschnitt des Instruments durch einen Schaft oder ein schaftartiges Bauteil von dem Betätigungsabschnitt bzw. dem Griffabschnitt getrennt ist. Der Begriff Schaft bzw. schaftartiges Bauteil bezeichnet hierbei ein Bauteil, dessen Abmessungen und Lage gegenüber dem Betätigungsabschnittl (z.B. Griffstück) auch während einer Betätigung des chirurgischen Instruments im Wesentlichen unveränderlich ist. Eine axiale Verschiebung entlang der Achse des Schafts oder schaftartigen Bauteils bzw. eine Verdrehung um diese Achse herum ist dabei zulässig, nicht jedoch eine wesentliche Verschiebung quer zu dieser Achse oder eine Verdrehung gegenüber dieser Achse in der Weise, dass sich die beiden Enden des Bauteils wesentlich von dieser Achse entfernen. Vorzugsweise ist die Länge eines Schafts oder schaftartigen Bauteils größer als dessen beide anderen Abmessungen (Breite, Tiefe) und es ist weiter vorzugsweise schlank ausgebildet. Der Schaft bzw. das schaftartige Bauteil muss dabei nicht rund, geschlossen, rohrförmig oder dünnwandig sein. Entscheidend ist hierbei, dass es sich um ein Instrument handelt, welches nicht wie eine gewöhnliche Schere einen Drehpunkt aufweist, um welchen sich alle wesentlichen Bestandteile des Instruments drehen, sondern dass die Kraft zum Öffnen und Schließen des Maulteils über eine relative axiale Bewegung eines Bauteils gegenüber dem Schaft übertragen wird.

Der Funktionsabschnitt bzw. Wirkabschnitt ist in dieser Anmeldung der Bereich des chirurgischen Rohrschaft-Instruments, an dem dessen eigentliche Funktion ausgeführt wird. Bei einem Nadelhalter ist es der Bereich, der die Nadel greift und hält, also die distalen Bereiche der Branchen. Bei einer Schere ist es der Bereich, der das Gewebe oder etwas anderes durchtrennt, also der Bereich, an dem die beiden aneinander vorbei gleitenden Scherkanten ausgebildet sind. Bei einem Clipapplikator ist es der Bereich, in dem der Clip zunächst gehalten wird, währen er durch den Chirurgen an die richtige Stelle und in die richtige Lage gebracht wird, und in dem der Clip anschließend appliziert wird, d.h. verpresst wird. Bei anderen Instrumenten ist die Definition des Funktionsabschnitts bzw. Wirkabschnitts entsprechend anzuwenden.

Der Wirkbereich ist der Bereich einer einzelnen Branche, an dem diese die bestimmungsgemäße Funktion des Instruments bewirkt, also bei einem Nadelhalter ein Greifbereich, bei einer Schere eine Scherkante und bei einem Clipapplikator ein Anlagebereich des Clips.

Ein Schließen bzw. ein Schließvorgang des Maulteils bedeutet hier, dass sich die Wirkbereiche der Branchen während des Schließvorgangs vorwiegend aufeinander zu bewegen. Im Falle einer Schere bewegen sich die Wirkbereiche der Branchen, also die Scherkanten, im späteren Verlauf des Schließvorgangs aneinander vorbei und dadurch wieder auseinander, trotzdem wird dieser ganze Vorgang als Schließvorgang des Maulteils bezeichnet. Allgemein gesagt bezeichnet ein Schließvorgang die Durchführung der dem Instrument zugewiesenen Funktion wie beispielsweise ein Greifen von Gewebe oder bspw. einer Nadel, ein Schneiden von Gewebe oder anderen Gegenständen, ein Applizieren eines Clips oder ein Aufspreizen von Gewebe oder anderen Gegenständen wie bspw. einem Clip. Ein sich anschließendes Öffnen bzw. ein sich anschließender Öffnungsvorgang bezeichnet dann allgemein die Rückkehr der Branchen, also auch der Wirkbereiche bzw. der distalen Bereiche der Branchen des Maulteils des jeweiligen Instruments, in ihre Ausgangsposition. Bei einem chirurgischen Spreizer ist die Zuordnung genau anders herum, da er seine Aufgabe ausübt, wenn sich die Branchen im Wesentlichen voneinander entfernen, also einen Öffnungsvorgang durchführen, wohingegen ein Zurückkehren der Branchen in die Ausgangsposition einem Schließvorgang entspricht. Trotzdem gibt es auch bei einem solchen Instrument einen klaren Öffnungsvorgang und einen klaren Schließvorgang.

### Allgemeine Beschreibung der Erfindung

Gemäß einem ersten Aspekt der vorliegenden Erfindung weist ein Maulteil für ein chirurgisches Rohrschaft-Instrument eine erste Branche mit einem ersten Wirkbereich, und eine zweite Branche mit einem zweiten Wirkbereich auf. Die erste Branche und/oder die zweite Branche verfügt über je ein Kulissenelement und die Branchen sind in axialer Richtung gehalten. Zudem ist ein Nockenträgerbauteil vorgesehen, welches relativ zu dem mindestens einen Kulissenelement in axialer Richtung verschiebbar ist, wobei das mindestens eine Kulissenelement und das Nockenträgerbauteil im Wesentlichen flächig ausgebildet sind und im Wesentlichen schichtartig übereinander angeordnet sind.

Mit dem erfindungsgemäßen Maulteil ist es möglich, die Kulissen und das Nockenträgerbauteil derart auszubilden und anzuordnen, dass die sich daraus ergebende Baugruppe in einem Bereich abseits des Wirkabschnitts des Maulteils zumindest in eine Richtung quer zur Längsachse des Maulteils eine deutlich geringere Abmessung als im Bereich des Wirkabschnitts des Maulteils aufweisen. Auf diese Weise ist es möglich, einen chirurgischen Clip von hinten, also von proximal in den Wirkabschnitt des Maulteils einzuführen. Wenn ein solcher Clip anschließend durch Verpressen appliziert wird und dann seitlich oder nach distal aus dem Wirkabschnitt des Maulteils entfernt wird (zumeist indem das Rohrschaft-Instrument zurück gezogen wird), kann ein weiterer Clip mit einer geeigneten Zuführeinrichtung aus einem Magazin von proximal in den Wirkabschnitt des Maulteils eingeführt werden. Zuführeinrichtungen für reguläre chirurgische Clips sind im Stand der Technik einige bekannt. Das erfindungsgemäße Maulteil aber ermöglicht es sogar, Doppelstegclips von proximal in den Wirkabschnitt des Maulteils einzuführen. Dies gilt auch für Rohrschaft-Instrumente mit einem besonders kleinem Außendurchmesser von ca. 5mm. Im Stand der Technik ist solche ein Maulteil nicht bekannt, da bei derartigen Abmessungen die Bauteile sehr filigran ausgebildet sein müssen und damit die Übertragung der erforderlichen Kräfte nicht mehr möglich ist. Erst die Verwendung von Kulissenelementen zum Steuern des Öffnungsvorgangs und insbesondere des Schließvorgangs und die geschichtete Anordnung dieser Kulissenelemente ermöglicht es, Doppelstegclips derart zuzuführen. Für andere Anwendungen als Clipapplikatoren oder Clipapplikatoren mit größerem Schaftdurchmesser hat das erfindungsgemäße Maulteil den Vorteil, dass erheblich größere Kräfte an den Wirkabschnitt übertragen werden können, als dies im Stand der Technik bei jeweils gleichem Schaftdurchmesser der Fall ist.

Um eine Relativbewegung zwischen dem mindestens einen Kulissenelement und dem Nockenträgerbauteil zu bewirken, muss zumindest ein Bauteil axial gehalten sein. Dies kann entweder durch ein gesondertes Haltebauteil erfolgen, oder aber ein Element des Rohrschafts eines Rohrschaftinstruments übernimmt diese Funktion, beispielsweise die Schaftwand. Das Haltebauteil kann auch einen Teil des Rohrschafts bezeichnen, der dafür ausgebildet ist, die axiale Lagerung des jeweiligen Kulissenelements zu gewährleisten. Dies gilt auch für alle weiteren Ausführungsformen des hier beschriebenen Maulteils.

Gemäß der vorliegenden Erfindung weisen das Nockenträgerbauteil und mindestens ein Kulissenelement mindestens einen Bereich auf, in dem sie eine Hinterschneidung ausbilden. Auf diese Weise ist ein Abheben des Kulissenelements von dem Nockenträgerbauteil verhindert. Es ist demnach nicht mehr erforderlich, ein Bauteil vorzusehen, welches ein Abheben der Bauteile voneinander verhindert, was zu einer Blockierung des Raumes führen würde, durch welchen ein Clip bzw. Doppelstegclip von proximal in den Wirkabschnitt des Maulteils eingeführt werden soll. Dies verringert einerseits die Anzahl an erforderlichen Bauteilen und vergrößert andererseits den frei verfügbaren Raum. Bevorzugt ist mindestens ein Bereich einer Hinterschneidung über den gesamten Bewegungsbereich des Kulissenelements zu dem Nockenträgerbauteil von einer vollständig geöffneten Stellung zu einer vollständig geschlossenen Stellung des Maulteils vorhanden. In diesem Fall kann ein Abheben über den gesamten Bewegungsbereich hinweg durch die Hinterschneidung verhindert werden. Ansonsten reicht es auch aus, wenn ein Abheben der Bauteile voneinander nur dann durch die Hinterschneidung verhindert wird, wenn sich kein Clip in dem Durchgangsbereich befindet, da dieser ansonsten ein Abheben der Bauteile voneinander verhindern kann.

Gemäß der vorliegenden Erfindung trägt das Nockenträgerbauteil wenigstens eine Nocke, die wenigstens über einen Teil der axialen Verschiebung des Nockenträgerbauteils zu dem Kulissenelement an einer Kulissenbahn anliegt, welche an dem Kulissenelement ausgebildet ist. Die Nocke weist vorzugsweise auf der Seite ihres axialen Querschnitts, welche der Kulissenbahn zugewandt ist, im Wesentlichen eine Z-Form, eine S-Form oder eine Kombination daraus auf. In diesem Fall bildet die Nocke einen Bauch und eine Kehle aus, wobei in die Kehle ein Teil des Kulissenelements eindringen kann, um mit dem Bauch eine Hinterschneidung auszubilden. Auf diese Weise kann ein Abheben des Nockenträgerbauteils von dem Kulissenelement verhindert werden. Selbstverständlich kann auch ein Abheben des Kulissenelements von dem Nockenträgerbauteil in der gleichen Weise verhindert werden.

Gemäß der vorliegenden Erfindung trägt das Nockenträgerbauteil wenigstens eine Nocke, die wenigstens über einen Teil der axialen Verschiebung des Nockenträgerbauteils zu dem Kulissenelement an einer Kulissenbahn anliegt, welche an dem Kulissenelement ausgebildet ist. Die Kulissenbahn weist vorzugsweise auf der Seite ihres axialen Querschnitts, welche der Nocke zugewandt ist, im Wesentlichen eine Z-Form, eine S-Form oder eine Kombination daraus auf. Mit diesem Aufbau kann ein Abheben des Nockenträgerbauteils und des Kulissenelements voneinander über den Bereich der gegenseitigen Bewegung verhindert werden, in dem eine gegenseitige Hinterschneidung der beiden Bauteile ausgebildet ist. Für die Hinterschneidung ist es sowohl für die Nocke als auch das Kulissenelement vorteilhaft, wenn der Bauch und die zugehörige Kehle des Bauteils zusammen eine Z-Form, eine S-Form oder eine Kombination daraus ausbilden. Weiter vorteilhaft ist es, wenn die beiden Querschnittsformen von Nocke und Kulissenbahn sich im Wesentlichen entsprechen. In diesem Fall kann die Neigung zur gegenseitigen Selbsthemmung der beiden Bauteile vermindert oder gar beseitigt werden.

Gemäß einer vorteilhaften Ausgestaltung des ersten Aspekts der vorliegenden Erfindung weisen die Nocke und die Kulissenbahn an den jeweils gegenseitig zugewandten Seiten ihres Querschnitts im Wesentlichen eine S-Form auf. Die Nocke und die Kulissenbahn bilden auf diese Weise je einen Bauch und eine Kehle aus und der Bauch eines Bauteils steht jeweils in die Kehle des anderen Bauteils vor. Die S-Form ist insbesondere vorteilhaft, da diese in den Dimensionen, welche die hier beschriebenen Bauteile aufweisen, leichter herzustellen ist und während der Montage nicht so anfällig wie die Z-Form ist. Dasselbe gilt aber auch für andere mögliche Formen wie zum Beispiel eine Stufenform, also eine mit rechten Winkeln ausgebildeter Bauch und eine entsprechende Kehle.

Gemäß einer weiteren vorteilhaften Ausgestaltung des ersten Aspekts der vorliegenden Erfindung ist zwischen Bauch und Kehle der Nocke und/oder der Kulissenbahn ein gerader Abschnitt ausgebildet. An diesem geraden Abschnitt des Querschnitts findet im Wesentlichen die Anlage der beiden Bauteile aneinander statt. In Erstreckungsrichtung der Nocke und/oder der Kulissenbahn muss dieser Abschnitt nicht gerade ausgebildet sein. In dieser Richtung ist vielmehr davon auszugehen, dass eine Krümmung der Nocke und/oder der Kulissenbahn vorliegt. Dieser gerade Abschnitt der Nocke und/oder der Kulissenbahn ist vorzugsweise gegenüber der Öffnungs-/Schließrichtung des Maulteils geneigt, weiter vorzugsweise mehr als 7°, insbesondere weniger als 20°. Wenn die beiden Bauteile aneinander anliegen, wird eine Kraft erzeugt, die quer zur Längsachse des Maulteils verläuft. Dies ist auch erforderlich, um einen Öffnungs- bzw. Schließvorgang des Maulteils steuern zu können. Wenn eine Neigung an dem Anlageabschnitt der beiden Bauteile ausgebildet ist, erzeugt die eingeleitete Querkraft eine resultierende Kraft, welche die beiden Bauteile zueinander hin drängt. Dies verhindert ein Abheben der beiden Bauteile voneinander. Ab einem Winkel von 7° ist bei den Dimensionen der Bauteile eine Herstellung der Bauteile vereinfacht, da diese dann weniger filigran ausgebildet sein müssen. Oberhalb eines Winkels von 20° erhöht sich die Reibung übermäßig, ohne dass sich daraus ein besonderer Vorteil ergibt.

Gemäß noch einer weiteren vorteilhaften Ausgestaltung des ersten Aspekts der vorliegenden Erfindung ist die Krümmung des Bauchs der Kulissenbahn größer als die Krümmung der Kehle der Kulissenbahn und/oder die Krümmung des Bauchs der Nocke ist größer als die Krümmung der Kehle der Nocke. Vereinfachend kann man sagen, dass die Kehle immer ein wenig größer als der darin aufgenommene Bauch ist. Mit dem Begriff Krümmung ist hier die durchschnittliche Krümmung des jeweiligen Bauteils gemeint. Die Krümmung kann sich entlang der Kehle oder dem Bauch natürlich verändern, sie kann sogar Sprünge aufweisen (d.h. in der Kehle oder dem Bauch ist ein Knick ausgebildet), aber im vorliegenden Fall ist zumindest in dem Bereich, in dem sich Kehle und Bauch berühren, die Krümmung des Bauches größer als die der Kehle. Größere Krümmung bedeutet kleinerer Radius, was dazu führt, dass entlang eines Querschnitts durch die Nocke und die Kulissenbahn nur maximal ein Anlagebereich zwischen Nocke und Kulissenbahn ausgebildet ist. Wenn die Krümmung der Kehle größer als die des darin aufgenommenen Bauches wäre, würden sich zwei Anlagebereiche ausbilden, was zu einer unnötig erhöhten Reibung der beiden Bauteile aneinander führen würde. Außerdem würde dies dazu führen, dass der Bauch die jeweilige Kehle aufspreizt, was zu einer Verformung des anderen Bauchs führen kann oder sogar zu eine Rissbildung entlang der Kehle.

Weiter vorteilhaft ist die Krümmung des Bauchs der Kulissenbahn größer als die Krümmung der Kehle der Nocke und/oder die Krümmung des Bauchs der Nocke ist größer als die Krümmung der Kehle der Kulissenbahn ist.

Gemäß einer anderen vorteilhaften Ausgestaltung des ersten Aspekts der vorliegenden Erfindung ist die Richtung der Krafteinleitung von der Nocke in die Kulissenbahn gegenüber der Öffnungs-/Schließrichtung des Maulteils geneigt ist, vorzugsweise in einem Bereich von bis zu 20°. Dies ist gleichbedeutend damit, dass der Kontaktpunkt zwischen Nocke und Kulissenbahn in einem Bereich ausgebildet, der gegenüber der Öffnungs-/Schließrichtung des Maulteils geneigt ist, vorzugsweise bis zu 20°. Wie dies vorstehend beschrieben ist, bildet sich bei der beschriebenen Krümmung von Bauch und Kehle der Bauteile nur ein Anlageabschnitt aus, der, wenn ein gewisser Unterschied in den beiden relevanten Krümmungen vorhanden ist, auch als Kontaktpunkt bezeichnet werden kann. In einer theoretischen Betrachtung bildet sich auch bei infinitesimalem Unterschied in der Krümmung der beiden betroffenen Bauteile nur ein Kontaktpunkt aus, in der Praxis hingegen führt die Elastizität der Bauteile dazu, dass sich ein definierter Kontaktpunkt erst ausbildet, wenn der Unterschied in der Krümmung einen gewissen Unterschied erreicht.

Gemäß einer vorteilhaften Ausgestaltung des ersten Aspekts der vorliegenden Erfindung ist die Oberfläche der Nocke im Bereich des Kontaktpunkts mit der Kulissenbahn der Mittelachse des Nockenträgerbauteils zugewandt. Mit einer solchen Ausbildung weisen die im Bereich der Kehle und des Bauches übertragenen Kräfte eine Richtung auf, die im Wesentlichen zu der Mittelachse des Nockenträgerbauteils hin verläuft. Auf diese Weise werden nur relativ geringe Torsionsmomente erzeugt. Große Torsionsmomente können bei den filigranen Bauteilen zu einer Verwindung derselben führen, welche das Maulteil beschädigen könnte.

Gemäß einer vorteilhaften Ausgestaltung des ersten Aspekts der vorliegenden Erfindung erstreckt sich mindestens ein Kulissenelement im Wesentlichen über die gesamte Breite des Nockenträgerbauteils, sodass die jeweilige Nocke des Nockenträgerbauteils im Wesentlichen durch das entsprechende Kulissenelement bedeckt ist. Für die Öffnungs- und Schließbewegung des Maulteils ist eine seitliche Anlage von Nocke und Kulissenbahn entscheidend. Für eine bessere gegenseitige Führung von Kulissenelement und Nockenträgerbauteil kann es vorteilhaft sein, wenn das Kulissenelement die Nocke bedeckt. Auf diese Weise können auch die sich möglicherweise ergebenden Torsionskräfte noch besser übertragen werden, sodass es zu keiner Verwindung des Nockenträgerbauteils kommt.

Gemäß einer vorteilhaften Ausgestaltung des ersten Aspekts der vorliegenden Erfindung weist mindestens ein Kulissenelement wenigstens zwei Kulissenbahnen auf, welche mit einer entsprechenden Anzahl von Nocken an dem Nockenträger wenigstens über einen Teil der axialen Verschiebung der beiden Bauteile zueinander von einer vollständig geöffneten Stellung zu einer vollständig geschlossenen Stellung des Maulteils je eine Hinterschneidung ausbilden, wobei Nockenträgerbauteil und Kulissenelement durch die Hinterschneidungen ausschließlich zusammenbaubar sind, indem ineinander eindrehbar sind. Der eindrehende Zusammenbau bezieht sich hierbei auf eine Drehung eines Bauteils gegenüber dem anderen Bauteil in der Ebene, in der das Öffnen und Schließen des Maulteils erfolgt. Zumindest eines der Bauteile kann dabei so elastisch sein, dass es während des Drehens zunächst an einer Stelle in Anlage an das andere Bauteil gerät und dann elastisch gebogen wird, bevor es an einer zweiten Stelle in die Hinterschneidung gelangt. Danach kann es sich elastisch zurück verformen.

Alternativ dazu kann auch ein Zusammenbau mit parallelen Längsachsen der Bauteile erfolgen. Dazu bedarf es einer Stellung der betroffenen Bauteile zueinander, in der keine Hinterschneidungen ausgebildet sind. In dieser Stellung werden die Bauteile aneinander herangeführt und anschließend in axialer Richtung verschoben, um Hinterschneidungen herzustellen. Besonders vorteilhaft ist die Montagestellung außerhalb des Arbeitsbereichs der Bauteile, sodass während des Betriebs trotzdem immer eine Hinterschneidung zwischen den betroffenen Bauteilen vorliegt.

Durch die vorstehende Ausbildung von mindestens einer Hinterschneidung über den gesamten gegenseitigen Bewegungsbereich des Kulissenelements und des Nockenträgerbauteils können diese beiden Bauteile nicht einfach mit im Wesentlichen parallelen Bauteillängsachsen aufeinander gesetzt werden. Aufeinandergesetzt bezieht sich hierbei auf den schichtartigen Aufbau, wobei die Richtung eines Aufsetzens rechtwinklig bzw. normal auf die Fläche der einzelnen Schichten steht.

Da das Nockenträgerbauteil eben auch mindestens eine Nocke aufweist und ein Kulissenelement in vielen Ausführungen auch auf beiden Seiten Kulissenbahnen ausgebildet hat, können diese beiden Bauteile auch nicht einfach seitlich zueinander gebracht werden.

Der komplexe Aufbau führt unter anderem auch zu einem besonderen Zusammenbau des Maulteils. Ein Kulissenbauteil und das Nockenträgerbauteil werden in zwei parallelen Ebenen angeordnet, wobei deren Bauteillängsachsen einen bestimmten Winkel zueinander innerhalb der parallelen Ebenen einnehmen. Nun werden die beiden Bauteile aneinander herangeführt, bis sie in Kontakt kommen. In diesem Zustand bilden die beiden Bauteile eine Art schiefes Kreuz oder Andreaskreuz. In diesem Zustand werden nun die beiden Bauteile so zueinander verdreht, dass ihre Bauteillängsachsen danach im Wesentlichen dieselbe Richtung aufweisen. Dieser Vorgang wird hier als Eindrehen bezeichnet und der Zusammenbau als eindrehender Zusammenbau. Mit einem solchen eindrehenden Zusammenbau ist es nämlich unmöglich, dass sich das Maulteil, wenn es einmal in einen Schaft eines Rohrschaftinstruments eingebaut ist, in seine einzelnen Bauteile zerlegt. Außerdem ist kein gesondertes Bauteil erforderlich, um den Zusammenhalt der Bauteile zu gewährleisten. Ist das Nockenträgerbauteil beispielsweise mit einer Schnappverbindung wie einer Schalbenschwanzverbindung an einem Vorschubelement des Rohrschaftinstruments befestigbar und sind die Kulissenbauteile beispielsweise mit elastisch gelagerten Vorsprüngen versehen, die in entsprechende Aussparungen in dem Rohrschaft eines Rohrschaftinstruments einschnappen können, ist somit ein vollkommen werkzeugfreier und montagematerialfreier Zusammenbau des Maulteils und Anbau desselben an einen Rohrschaft eines Rohrschaftinstruments möglich.

Gemäß wieder einer vorteilhaften Ausgestaltung des ersten Aspekts der vorliegenden Erfindung befinden sich das Kulissenelement und das Nockenträgerbauteil nach einem eindrehenden Zusammenbau in einer Montagestellung, die sich außerhalb der relativen axialen Verschiebung des Kulissenelements und des Nockenträgerbauteils zueinander von einer vollständig geöffneten Stellung zu einer vollständig geschlossenen Stellung des Maulteils befindet. Die Position der Bauteile zueinander wird dann während der Montage an einem Rohrschaft verändert, sodass die Montagestellung in eine Ausgangsstellung des Maulteils für einen Schließvorgang (bzw. Öffnungsvorgang bei einem Spreizinstrument o.ä.) überführt wird. Bei den vorstehend beschriebenen Schnappverbindungen kann dies beispielsweise erreicht werden, indem die Verbindung zwischen Kulissenelementen und Rohrschaftwand zuerst hergestellt wird, und erst ein wenig später, d.h. nach einer gewissen Wegstrecke, die Verbindung zwischen Nockenträgerbauteil und Vorschubelement hergestellt wird. Dann findet während der Montage eine Verschiebung zwischen den Kulissenelementen und dem Nockenträgerbauteil statt. Üblicherweise wird ein Maulteil an einem Rohrschaft montiert, indem die Maulteilbaugruppe bestehend aus dem Nockenträgerbauteil und den Kulissenelementen von distal in das vordere Ende des Rohrschafts eingeführt werden.

Gemäß einem zweiten Aspekt der vorliegenden Erfindung weist ein Maulteil für ein chirurgisches Rohrschaft-Instrument eine erste Branche mit einem ersten Wirkbereich und eine zweite Branche mit einem zweiten Wirkbereich auf. Die erste Branche und die zweite Branche verfügen über je ein Kulissenelement und die Branchen sind in axialer Richtung gehalten. Ein Nockenträgerbauteil ist vorgesehen, welches relativ zu den Kulissenelementen in axialer Richtung verschiebbar ist, wobei die Kulissenelemente im Wesentlichen flächig ausgebildet sind und im Wesentlichen schichtartig übereinander angeordnet sind und das Nockenträgerbauteil im Wesentlichen eine hohlen Querschnitt aufweist, in dem die Kulissenelemente angeordnet sind.

Im Gegensatz zu dem ersten Aspekt der vorliegenden Erfindung weist der zweite Aspekt sogenannte innenliegende Kulissenelemente auf. Diese werden so bezeichnet, da sie im montierten Zustand von dem Nockenträgerbauteil umgeben sind. Das generelle Prinzip der Maulteilsteuerung entspricht aber dem des ersten Aspekts der vorliegenden Erfindung. Auch bei dieser Ausführung kann ein Haltebauteil vorgesehen sein oder die Kulissenelemente sind durch ein Element eines Rohrschafts gehalten.

Gemäß einer vorteilhaften Ausgestaltung des zweiten Aspekts der vorliegenden Erfindung sind das Kulissenbauteil und das Nockenträgerbauteil von einer Montagestellung durch eine axiale Bewegung relativ zueinander in eine vollständig geöffnete Stellung bringbar.

Die vollständig geöffnete Stellung entspricht Grundstellung bzw. Nullstellung, über die hinaus im montierten Zustand keine Relativverschiebung zwischen den beiden Bauteilen möglich ist.

Erfindungsgemäß wird folglich ein Maulteil mit den vorstehend genannten Merkmalen oder individuell beanspruchbaren Merkmalskombinationen vorgeschlagen, das der Schwenkachsenlosen/Schwenkbolzenlosen Bauart angehört, also keine gegenständliche Schwenkachse aufweist.

Gemäß einem dritten Aspekt der vorliegenden Erfindung ist ein Verfahren zum Zusammenbau einer Branche und eines Nockenträgerbauteils für ein vorstehend beschriebenes Maulteil offenbart, welches die folgenden Schritte aufweist. Zunächst wird eine Branche und eine Nockenträgerbauteil in einem Zustand bereit gestellt, in dem ein Kulissenelement der Branche der zugehörigen Seite des Nockenträgerbauteils zugewandt ist. Der Begriff zugehörig bedeutet dabei, dass die Form und Lage der Kulissenbahn bzw. Kulissenbahnen der Branche und die Form und Lage der Nocke bzw.

Nocken des Nockenträgerbauteils so aufeinander abgestimmt sind, dass sie einen Öffnungs- und Schließvorgang der beiden Bauteile zueinander bewirken können. Dabei sind die Branche und das Nockenträgerbauteil in zwei parallelen Ebenen angeordnet, die voneinander beabstandet sind, und die Längsachsen der beiden Bauteile nehmen einen bestimmten Winkel zwischen sich ein. Dieser Winkel hängt wiederum von Form und Lage der Nocke(n) und Kulissenbahn(en) ab. Die Nocke(n) und die Kulissenbahn(en) stehen von den jeweiligen Grundflächen des Nockenträgerbauteils bzw. des Kulissenelements der Branche hervor. Der bestimmte Winkel ist demnach so gewählt, dass es keine Überlagerung einer Nocke mit einem eine Kulissenbahn ausbildenden Bereich gibt. Anschließend werden die Branche und des Nockenträgerbauteil aneinander herangeführt, bis diese in Kontakt gelangen. Da es keine Überlagerung zwischen einer Nocke und einem eine Kulissenbahn ausbildenden Bereich gibt, erfolgt ein Kontakt der beiden Grundflächen von Kulissenelement und Nockenträgerbauteil. Nun werden die Branche und das Nockenträgerbauteil relativ zueinander in der durch die ihre Kontaktfläche bestimmten Ebene in der Weise gedreht, dass sich der Winkel zwischen ihren Längsachsen verringert. Genau genommen gibt es keinen Schnittpunkt zwischen den Längsachsen der Beiden Bauteile. Der vorstehen genannte Winkel ist der Winkel, den die Längsachsen der beiden Bauteile erzeugen, wenn sie parallel in die Kontaktebene von Kulissenelement und Nockenträgerbauteil projiziert werden. Diese Ebene wird durch die Grundflächen der beiden Bauteile bestimmt. Die Drehung der beiden Bauteile gegeneinander wird fortgeführt, bis mindestens je eine Nocke des Nockenträgerbauteils beiderseits des Kreuzungspunkts der beiden Längsachsen in Kontakt mit einer Kulissenbahn gelangt. Auf diese Weise wird eine weitere Drehung verhindert. Während der Drehung kann auch eine Verschiebung eines der Bauteile oder beider Bauteile entlang ihrer Längsachsen erfolgen, sodass die Bewegung der Bauteile zueinander nicht rein rotatorisch ist. Es ist aber entscheidend, dass ein rotatorischer Anteil in der gegenseitigen Bewegung der beiden Bauteile enthalten ist. Außerdem kann während der Bewegung vorzugsweise mindestens eine Hinterschneidung zwischen einer Nocke und einem Kulissenelement ausgebildet werden.

Vorzugsweise sind dabei das mindestens eine Kulissenelement und das Nockenträgerbauteil im Wesentlichen flächig ausgebildet und im Wesentlichen schichtartig übereinander angeordnet.

Mit diesem Verfahren wird ein besonders einfacher, schneller, werkzeugfreier und kostengünstiger Zusammenbau des Maulteils ermöglicht. Die so gebildete Baugruppe des Maulteils lässt sich leicht weiter verarbeiten.

Gemäß eines vorzugsweisen Aspekts der vorliegenden Erfindung ist ein Verfahren zum Zusammenbau eines chirurgischen Rohrschaft-Instruments offenbart. Das Rohrschaftinstrument weist ein vorstehend beschriebenes Maulteil, ein Schaftbauteil und einen Betätigungsstab auf. Der Zusammenbau beginnt mit einem Bereitstellen einer Branche und eines Nockenträgerbauteils in einem Zustand, in dem ein Kulissenelement der einen Branche der zugehörigen Seite des Nockenträgerbauteils zugewandt ist. Die eine Branche und das Nockenträgerbauteil sind in zwei parallelen Ebenen angeordnet, die voneinander beabstandet sind, und die Längsachsen der beiden Bauteile einen bestimmten Winkel zwischen sich ein. Dies erfolgt in Übereinstimmung mit dem dritten Aspekt der vorliegenden Erfindung, wobei auch die dort gegebene Erklärung zu den Winkeln bei diesem Aspekt Anwendung findet. Anschließend werden die eine Branche und das Nockenträgerbauteil aneinander herangeführt, bis diese in Kontakt gelangen. Danach werden die eine Branche und das Nockenträgerbauteil relativ zueinander in der durch die ihre Kontaktfläche bestimmten Ebene in der Weise gedreht, dass sich der Winkel zwischen ihren Längsachsen verringert, bis mindestens je eine Nocke des Nockenträgerbauteils beiderseits des Kreuzungspunkts der beiden Längsachsen in Kontakt mit einer Kulissenbahn der einen Branche gelangt. Das Verfahren weist zudem noch ein Anbringen einer anderen Branche an der gegenüberliegenden Seite des Nockenträgerbauteils auf. Allerdings kann dies auch vor den vorgenannten Verfahrensschritten geschehen, sodass die andre Branche auch die zuerst an dem Nockenträgerbauteil montierte Branche sein kann. Diese andere Branche kann entweder in gleicher Weise an dem Nockenträgerbauteil montiert werden, wie dies vorstehend für die eine Branche beschrieben ist, sie kann aber auch gänzlich anders montiert werden. Wenn die andere Branche beispielsweise unbeweglich gehalten sein soll, kann sie einfach auf das Nockenträgerbauteil aufgesetzt werden, ohne dass eine Drehbewegung zwischen der anderen Branche und dem Nockenträgerbauteil erforderlich ist. In diesem Fall kann Das Nockenträgerbauteil beispielsweise zwei oder mehr pilzförmige Vorsprünge aufweisen, die jeweils in ein Rundloch in dem Kulissenelement der anderen Branche eingesetzt werden können, von denen sich in axialer Richtung ein Langloch mit einer Breite erstreckt, der im Wesentlichen dem Durchmesser des Schaftes der pilzförmigen Vorsprünge entspricht. So ist eine axiale Relativbewegung zwischen dem Nockenträgerbauteil und der anderen Branche möglich, ohne dass sich eine laterale Bewegung der Bauteile zueinander ergibt. Es können aber auch andere bekannte Verbindungsmechanismen verwendet werden. Prinzipiell ist es auch möglich, dass gar keine andere Branche montiert wird, da diese beispielsweise an dem distalen Ende des Schaftbauteils ausgebildet ist. Nach dem Montieren des Maulteils wird dieses mit seinem proximalen Ende in ein distales Ende des Schaftbauteils eingeschoben. Dabei gelangen die eine Branche und/oder die andere Branche sowie das Nockenträgerbauteil zu unterschiedlichen Zeitpunkten mit einem von dem Schaftbauteil oder dem Betätigungsstab in Eingriff, sodass dadurch eine axiale Verschiebung zwischen dem Nockenträgerbauteil sowie der einen Branche und/oder der anderen Branche in eine Stellung erfolgt, die der vollständig geöffneten Stellung des Maulteils entspricht. Wenn die beiden Branchen in ähnlicher Weise an dem Nockenträgerbauteil montiert sind, ist es vorteilhaft, wenn diese auch in etwa gleichzeitig mit dem Schaftbauteil oder dem Betätigungsstab in Eingriff gelangen. Bevorzugt ist es hierbei, wenn die Branchen mit dem Schaftbauteil in Eingriff gelangen und das nockenträgerbauteil mit dem Betätigungsstab in Eingriff gelangt. Weiter vorzugsweise gelangt zuerst das Nockenträgerbauteil mit dem Betätigungsstab in Eingriff, wodurch eine weitere axiale Verschiebung des Nockenträgerbauteils nach proximal verhindert ist. Die Branche bzw. die Branchen können dann noch weiter nach proximal verschoben werden, bis diese mit dem Schaftbauteil in Eingriff gelangen. Eine Branche weist dazu vorzugsweise einen sich nach proximal erstreckenden elastischen Fortsatz auf, der das proximale Ende der Branche zu der Innenwand des Schaftbauteils hin drängt. an der der Innenwand des Schaftbauteils zugewandten Seite weist der Fortsatz wenigstens einen Vorsprung auf, der in eine Vertiefung bzw. Aussparung in dem Schaftbauteil eindringen kann, um so eine Verbindung zwischen der Branche und dem Schaftbauteil herzustellen. Nach einer vorteilhaften Weiterbildung ist auf der von der Innenwand des Schaftbauteils abgewandten Seite des Fortsatzes eine Fläche ausgebildet, die mit einer entsprechenden Fläche an dem Nockenträgerbauteil so zusammenwirkt, dass sie bei der Relativverschiebung von Branche und Nockenträgerbauteil durch bzw. während der Montage zu dem Zeitpunkt, zu dem der mindestens eine Vorsprung an dem Fortsatz der Branche in die Aussparung in dem Schaftbauteil eingedrungen und die Branche dadurch axial an dem Schaftbauteil fixiert ist, eine Hinterschneidung ausbilden, sodass sich der Fortsatz an der Branche nicht mehr so weit zu Achse des Schaftbauteils hin bewegen kann, dass der mindestens eine Vorsprung am Fortsatz der Branche vollständig aus der Aussparung in dem Schaftbauteil heraustreten kann. Da sich das Nockenträgerbauteil, wenn es erst einmal an dem Betätigungsstab befestigt wurde, nicht mehr so weit nach distal bewegen lässt, dass diese Hinterschneidung mit dem Fortsatz der Branche aufgegeben werden kann, ist somit eine Demontage des Maulteils von dem Schaftbauteil sicher verhindert.

Vorzugsweise sind das mindestens eine Kulissenelement und das Nockenträgerbauteil im Wesentlichen flächig ausgebildet und im Wesentlichen schichtartig übereinander angeordnet.

Mit dem beschriebenen Verfahren lässt sich ein Rohrschaft-Instrument schaffen, welches besonders einfach zusammen gebaut werden kann, welches nach einem Zusammenbau nicht wieder demontiert werden kann und bei welchem ein Abheben eines Kulissenelements von dem Nockenträgerbauteil sicher verhindert ist.

Alternativ zu der vorstehend beschriebenen Art des Zusammenbaus der beiden Kulissenelemente und des Nockenträgerbauteils gibt es noch andere Varianten. In einer ersten alternativen Variante sind die beiden Kulissenelemente in der Ebene, in der sie sich im Wesentlichen erstrecken, elastisch biegbar. Die Kulissenelemente können in diesem Fall in ihrer Erstreckungsebene gebogen werden, sodass keine Überlappungen zwischen den Kulissenbahnen und den Nocken des Nockenträgerbauteils vorhanden sind, und dann an das Nockenträgerbauteil herangeführt werden. Erst wenn das Kulissenelement an dem Nockenträgerbauteil anliegt, werden die Kulissenelemente in ihre Ausgangsform zurück entspannt, wobei sich dann die Hinterschneidungen zwischen den Kulissenbahnen und den Nocken ausbilden. In einer zweiten alternativen Variante sind die Kulissenelemente mit Unterbrechungen ausgestattet, die nur geringfügig größer als die zugehörigen Nocken sind. Diese Unterbrechungen sind so angeordnet, dass es eine relative Anordnung von Nockenträgerbauteil und Kulissenelement gibt, in der allen Nocken des Nockenträgerbauteils eine entsprechende Aussparung zugewandt ist. In dieser relativen Anordnung können das Kulissenelement und das Nockenträgerbauteil aneinander herangeführt werden, wobei die Nocken durch die Aussparungen in die Kulissenbahnen eindringen. Bei einer relativen axialen Verschiebung der beiden Bauteile zueinander werden dann Hinterschneidungen zwischen Kulissenbahnen und Nocken hergestellt. Vorteilhaft ist es, wenn die relative Anordnung für den Zusammenbau sich außerhalb eines Bereichs befindet, in dem sich die beiden Bauteile bei einer gewöhnlichen Betätigung zueinander bewegen. In einer dritten alternativen Variante sind die Kulissenelemente senkrecht zu ihrer Erstreckungsebene elastisch biegbar. Außerdem weißen die Kulissenelemente die vorstehend beschriebenen Aussparungen in den Kulissenbahnen auf, nur dass diese nicht in denselben Abständen wie denen der Nocken angeordnet sind. Bei dieser Variante wird ein Kulissenelement zum Zusammenbau mit dem Nockenträgerbauteil aus der Erstreckungsebene heraus gebogen, eine erste Nocke wird in einer ersten Kulissenbahn durch eine erste Aussparung eingefädelt. Anschließend werden Nockenträgerbauteil und Kulissenelement zueinander axial verschoben, bis eine zweite Nocke einer zweiten Aussparung gegenüber liegt. Nun wird diese zweite Nocke durch die zweite Aussparung in die zweite Kulissenbahn eingefädelt, wobei dieser Abschnitt der Kulissenbahn in seine Ausgangslage zurück gebogen wird. Gegebenenfalls werden noch weitere Nocken auf dieselbe Weise in die Kulissenbahn eingefädelt. Der Vorteil dieser Variante ist, dass es keine relative Stellung von Nockenträgerbauteil und Kulissenelement zueinander gibt, in der die beiden Bauteile demontiert werden können, ohne dass eine elastische Verformung auf das Kulissenelement aufgebracht werden muss. Diese Ausführungsform ist also vor einem versehentlichen Auseinanderfallen gesichert.

Weitere Vorteile und Merkmale der Erfindung sind dem Fachmann aus den beigefügten Figuren und der detaillierten Beschreibung der Ausführungsbeispiele ersichtlich.
- Fig. 1: zeigt eine isometrische Ansicht eines ersten Ausführungsbeispiels einer ersten Branche des Maulteils gemäß dem ersten Aspekt der vorliegenden Erfindung, wobei die Fig. 1A eine reguläre Stellung der ersten Branche und die Fig. 1B eine um 90° nach außen gekippte Stellung zeigt;
- Fig. 2: zeigt eine isometrische Ansicht eines ersten Ausführungsbeispiels einer zweiten Branche des Maulteils gemäß dem ersten Aspekt der vorliegenden Erfindung, wobei die Fig. 2A eine reguläre Stellung der zweiten Branche und die Fig. 2B eine um 90° nach außen gekippte Stellung zeigt;
- Fig. 3: zeigt eine isometrische Ansicht eines ersten Ausführungsbeispiels eines Nockenträgerbauteils des Maulteils gemäß dem ersten Aspekt der vorliegenden Erfindung, wobei die Fig. 3A eine Ansicht von proximal und die Fig. 3B eine Ansicht von distal zeigt;
- Fig. 4: zeigt eine isometrische Ansicht des Maulteils inklusive einem Teil eines Betätigungsstabs eines Rohrschaftinstruments gemäß den Fig. 1 bis 3;
- Fig. 5: zeigt eine Schnittansicht des Maulteils gemäß Fig. 4;
- Fig. 6: zeigt eine Draufsicht auf das Maulteil gemäß Fig. 4 während einer Montage eines ersten Maulteils;
- Fig. 7: zeigt eine isometrische Ansicht eines proximalen Endes eines zweiten Ausführungsbeispiels des Nockenträgerbauteils gemäß dem ersten Aspekt der vorliegenden Erfindung und des distalen Endes eines Betätigungsstabs eines Rohrschaftinstruments;
- Fig. 8: zeigt eine Schnittansicht eines zweiten Ausführungsbeispiels des Maulteils gemäß dem ersten Aspekt der vorliegenden Erfindung;
- Fig. 9: zeigt eine Schnittansicht eines dritten Ausführungsbeispiels des Maulteils gemäß dem ersten Aspekt der vorliegenden Offenbarung;
- Fig. 10: zeigt eine Schnittansicht eines vierten Ausführungsbeispiels des Maulteils gemäß dem ersten Aspekt der vorliegenden Erfindung;
- Fig. 11: zeigt eine Schnittansicht eines fünften Ausführungsbeispiels des Maulteils gemäß dem ersten Aspekt der vorliegenden Erfindung;
- Fig. 12: zeigt eine Schnittansicht eines sechsten Ausführungsbeispiels des Maulteils gemäß einem zweiten Aspekt der vorliegenden Erfindung;
- Fig. 13: zeigt eine Schnittfläche durch die Kulissenelemente und das Nockenträgerbauteil entsprechend Fig. 12; und
- Fig. 14: zeigt eine isometrische Ansicht des sechsten Ausführungsbeispiels eines Maulteils gemäß dem zweiten Aspekt der vorliegenden Erfindung.

Die Ausführungsbeispiele der verschiedenen Aspekte der vorliegenden Erfindung sind im Folgenden unter Bezugnahme auf die Figuren beschrieben.

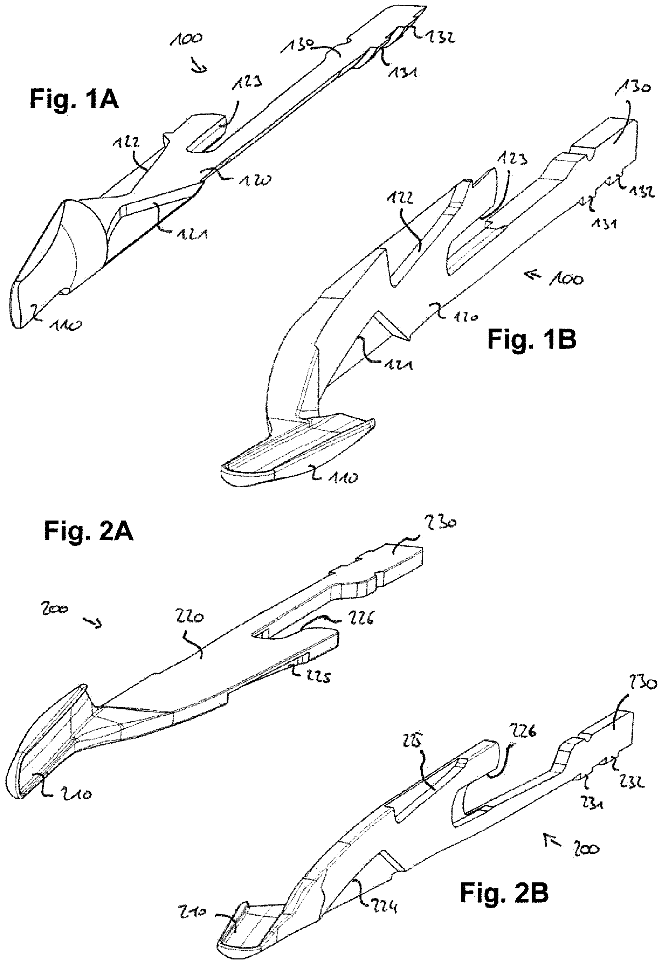

Unter Bezugnahme auf die Fig. 1 bis 7 ist im Folgenden ein erstes Ausführungsbeispiel des ersten Aspekts der vorliegenden Erfindung im Detail beschrieben.

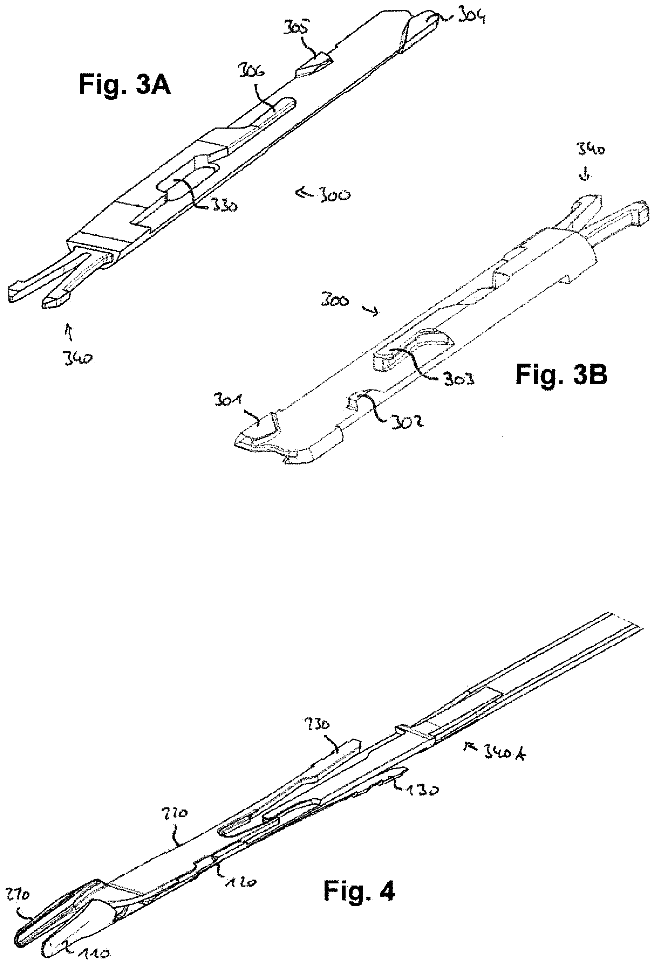

In Fig. 4 ist ein Maulteil 1 für ein chirurgisches Rohrschaft-Instrument gezeigt. Das Rohrschaftinstrument ist eine chirurgische Clipanlegezange für Doppelstegclips, die aufgrund ihres Herstellungsverfahrens auch Ringclips genannt werden. Im vorliegenden Fall ist die Clipanlegezange für das serielle Anlegen einer Vielzahl von Clips gestaltet. Solch eine Clipanlegezange wird als multifire Clipapplikator oder Clipapplikator der Mehrschussart bezeichnet. Bei diesem Clipapplikator ist ein Magazin von Doppelstegclips vorgesehen, von denen nach der Applikation eines Clips ein nächster Clip von proximal in das Maulteil 1 eingeführt wird, sodass die nächste Applikation sofort anschließend vorgenommen werden kann.

Das Maulteil weist eine erste Branche 100 mit einem ersten Wirkbereich 110 und eine zweite Branche 200 mit einem zweiten Wirkbereich 210 auf. Die erste Branche 100 und die zweite Branche 200 verfügen über je ein Kulissenelement 120, 220 und die Branchen 100, 200 sind in axialer Richtung gehalten. Ein Nockenträgerbauteil 300 ist relativ zu den beiden Kulissenelementen 120, 220 in axialer Richtung verschiebbar angeordnet und die beiden Kulissenelemente 120, 220 sowie das Nockenträgerbauteil 300 sind im Wesentlichen flächig ausgebildet und sind im Wesentlichen schichtartig übereinander angeordnet. Das Nockenträgerbauteil 300 und die Kulissenelemente 120, 220 weisen mehrere Bereiche auf, in denen sie eine Hinterschneidung ausbilden. Auf diese Weise ist ein Abheben der Kulissenelemente 120, 220 von dem Nockenträgerbauteil 300 sicher verhindert. Ein Bereich einer Hinterschneidung zwischen jedem Kulissenelement 120, 220 und dem Nockenträgerbauteil 300 bleibt über den gesamten Bewegungsbereich der Kulissenelemente 120, 220 zu dem Nockenträgerbauteil 300 von einer vollständig geöffneten Stellung, wie sie in Fig. 4 gezeigt ist, bis zu einer vollständig geschlossenen Stellung des Maulteils 1 erhalten. Dies bedeutet, dass über den gesamten Bewegungsbereich des jeweiligen Kulissenelements 120, 220 zu dem Nockenträgerbauteil 300 immer mindestens eine Hinterschneidung zwischen den beiden betroffenen Bauteilen ausgebildet ist, wobei zeitweise auch an mehreren Stellen Hinterschneidungen ausgebildet sein können und eine einzelne Hinterschneidung gegebenenfalls nur über einen gewissen Teilbereich der Relativbewegung existiert.

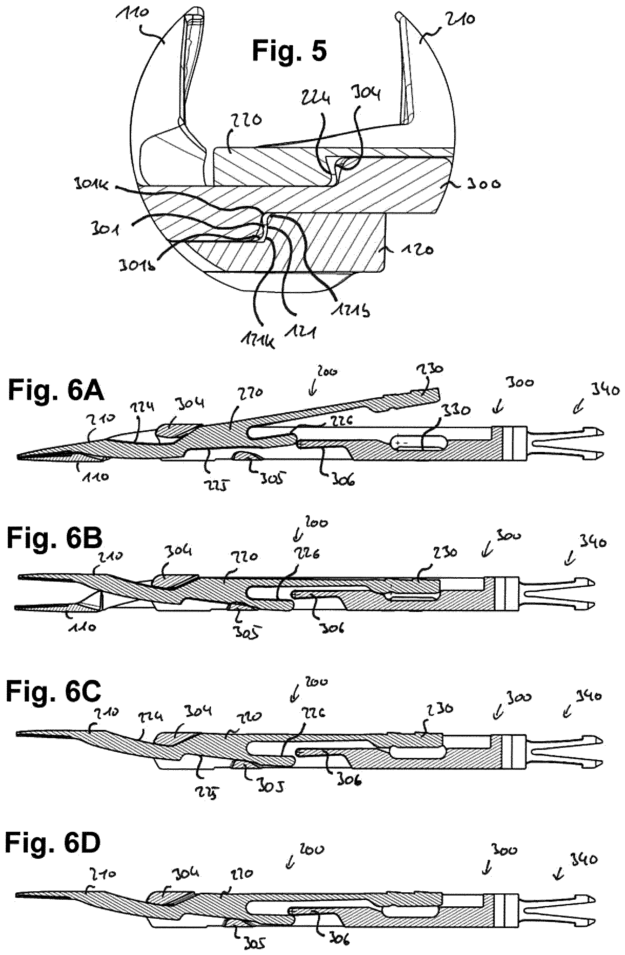

Das Nockenträgerbauteil 300 trägt bei diesem Ausführungsbeispiel sechs Nocken 301 bis 306. Jede dieser Nocken 301 bis 306 liegt über zumindest einen Teil der axialen Verschiebung des Nockenträgerbauteils 300 zu dem jeweiligen Kulissenelement 120, 220 an einer Kulissenbahn 121, 122, 123, 224, 225, 226 an, welche an dem Kulissenelement 120, 220 ausgebildet ist. Die Nocken 301 bis 306 weisen auf der jeweils der Kulissenbahn 121, 122, 123, 224, 225, 226 zugewandten Seite ihres axialen Querschnitts im Wesentlichen eine S-Form auf, wobei diese Form in den Fig. 1 und 2 aus Gründen der Übersichtlichkeit nicht gezeigt ist. Passend dazu sind die Kulissenbahnen 121, 122, 123, 224, 225, 226 auf der der jeweiligen Nocke 301 bis 306 zugewandten Seite ihres axialen Querschnitts im Wesentlichen ebenfalls eine S-Form auf. Auch diese Form ist in der Fig. 3 aus Gründen der Übersichtlichkeit nicht gezeigt. Dafür sind in Fig. 5 die Formen der Nocken 301, 304 und der zugehörigen Kulissenbahnen 121, 224 beispielhaft für alle Nocken und Kulissenbahnen dieses Ausführungsbeispiels gezeigt.

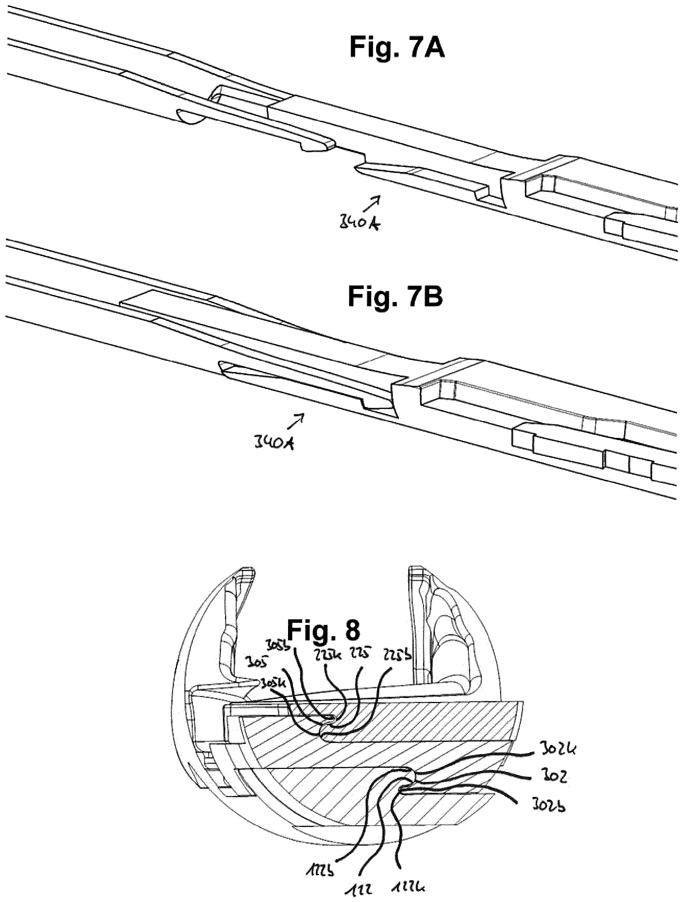

Wie dies aus Fig. 5 ersichtlich ist, bilden Nocken 301 bis 306 und Kulissenbahnen 121, 122, 123, 224, 225, 226 auf diese Weise je einen Bauch und eine Kehle aus. Detailliert gezeigt ist dies in Fig. 5 für die Kulissenbahn 121, welche einen Bauch 121b und eine Kehle 121k ausbildet, sowie für die zugehörige Nocke 301, welche einen Bauch 301b und eine Kehle 301k ausbildet. Der Bauch 301b ragt dabei in die Kehle 121k und der Bauch 121b ragt in die Kehle 301k, wobei auf diese Weise eine Hinterschneidung ausgebildet wird. Besonders anschaulich ist dies in Fig. 8 für ein zweites Ausführungsbeispiel gezeigt, welches später beschrieben ist.

Wie dies aus den Fig. 4 und 5 ersichtlich ist, erstreckt sich das Kulissenelement 220 im Wesentlichen über die gesamte Breite des Nockenträgerbauteils 300, sodass die Nocke 304 des Nockenträgerbauteils 300 im Wesentlichen durch das Kulissenelement 220 bedeckt ist.

Wie dies vorstehend bereits beschrieben ist, weist bei diesem Ausführungsbeispiel jedes Kulissenelement 120, 220 drei Kulissenbahnen 121, 122, 123 bzw. 224, 225, 226 auf, welche sich mit jeweils drei Nocken 301 bis 303 bzw. 304 bis 306 an dem Nockenträger 300 wenigstens über einen Teil der axialen Verschiebung der beiden Bauteile zueinander von einer vollständig geöffneten Stellung zu einer vollständig geschlossenen Stellung des Maulteils 1 in einer Hinterschneidung befinden. Dadurch ist das Nockenträgerbauteil 300 und jedes der beiden Kulissenelemente 120, 220 ausschließlich zusammenbaubar, indem ein Kulissenelement 120, 220 in das Nockenträgerbauteil 300 eindrehbar ist. Wie dies im Detail funktioniert, ist in Fig. 6 beschrieben. Im Folgenden ist der Zusammenbau für die Branche 100 gezeigt. Bei dem vorliegenden Ausführungsbeispiel erfolgt der Zusammenbau mit der Branche 200 in gleicher Weise.

Zunächst werden die Branche 100 und das Nockenträgerbauteil 300 in einem Zustand, in dem das Kulissenelement 120 der Branche 100 der zugehörigen Seite des Nockenträgerbauteils 300 zugewandt ist, bereit gestellt. Die Branche 100 und das Nockenträgerbauteil 300 sind dabei in zwei parallelen Ebenen angeordnet, die voneinander beabstandet sind, und die Längsachsen der beiden Bauteile 100, 300 nehmen einen Winkel von ungefähr 10° zwischen sich ein. Anschließend werden die Branche 100 und das Nockenträgerbauteil 300 aneinander herangeführt, bis diese in Kontakt gelangen, wie dies in Fig. 6A gezeigt ist. Üblicherweise wird dabei die Branche 100 auf das Nockenträgerbauteil 300 zu bewegt, es kann aber auch das Nockenträgerbauteil 300 auf die Branche 100 zu bewegt werden. Anschließend wird die Branche 100 relativ zu dem Nockenträgerbauteil 300 in der durch die ihre Kontaktfläche bestimmten Ebene in der Weise gedreht, dass sich der Winkel zwischen ihren Längsachsen verringert. Die Branche 100 wird so lange gedreht, bis die Kulissenbahn 224 in Anlage an die Nocke 304 gelangt und die Kulissenbahn 225 in Anlage an die Nocke 305 gelangt, wie dies in Fig. 6B gezeigt ist. Wie dies ebenfalls aus Fig. 6A ersichtlich ist, liegen die beiden Nocken 304, 305 und die beiden Kulissenbahnen 224, 225 auf gegenüberliegenden Seiten des Kreuzungspunkts der Längsachsen der beiden Bauteile 200, 300.

In der in Fig. 6B gezeigten Stellung befinden sich das Kulissenelement 220 und das Nockenträgerbauteil 300 nach einem eindrehenden Zusammenbau in einer Montagestellung. Diese Montagestellung befindet sich außerhalb der relativen axialen Verschiebung des Kulissenelements 220 und des Nockenträgerbauteils 300 zueinander von einer vollständig geöffneten Stellung zu einer vollständig geschlossenen Stellung des Maulteils 1 und ist dadurch gekennzeichnet, dass ein Fortsatz 130, 230, der an jeder Branche 100, 200 vorgesehen ist und gegenüber dem jeweiligen Kulissenelement 120, 220 lateral elastisch ist, d.h. sich elastisch auf einem Bogen von der Längsachse des Bauteils weg und zu diesem hin bewegen kann, mit seinem proximalen Ende in eine Aussparung 330 eintauchen kann, welche in dem Nockenträgerbauteil 300 ausgebildet ist. Taucht das proximale Ende des Fortsatzes 230 in diese Aussparung 330 ein, so sind die lateralen Vorsprünge 231, 232 des Fortsatzes 230 so weit zur Längsachse der Branche 200 hin verschoben, dass das so zusammengebaute Maulteil 1 in ein distales Ende eines Rohrschafts eines Rohrschaftinstruments eingeführt werden kann. Beim Einführen drängt das distale Ende des Fortsatzes 230 nach außen gegen die Wand des Rohrschafts. In der Rohrschaftwand sind zwei Aussparungen vorgesehen, die daran angepasst sind, die beiden Vorsprünge 231, 232 aufzunehmen. Gleiten die Vorsprünge 231, 232 in diese Aussparungen in der Rohrschaftwand, ist die Branche 200 relativ zu der Rohrschaftwand axial gehalten, sodass bei einem weiteren Einführen des Maulteils 1 in den Rohrschaft nur noch das Nockenträgerbauteil 300 bewegt wird. Hierbei wird davon ausgegangen, dass die andere Branche 100 einen vergleichbaren Aufbau wie die Branche 200 aufweist und deren Vorsprünge 131, 132 zur selben Zeit in entsprechende Aussparungen in der Rohrschaftwand einrückt. Dann wird die Schubstange von proximal in den Rohrschaft eingeschoben, bis die Spange 340 des Nockenträgerbauteils 300 in eine entsprechende Aufnahme in eine Aufnahme eingreift, die in die Schubstange ausgebildet ist. Die Schubstange wird in dem Rohrschaft angeordnet ist, um eine Betätigung des Maulteils 1 zu bewirken. Der Moment, in dem die Vorsprünge 231, 231 in die Aussparungen in der Rohrschaftwand eintauchen, ist in Fig. 6C gezeigt. In Fig. 6D ist erkennbar, dass eine Relativverschiebung zwischen Branche 200 und Nockenträgerbauteil 300 stattgefunden hat, sodass das distale Ende des Fortsatzes 230 nicht mehr in die Aussparung 330 eintauchen kann. Gleichzeitig kann, da das Nockenträgerbauteil 300 von der Schubstange axial gehalten ist, dieses nicht mehr weiter als in Fig. 6D gezeigt nach distal bewegt werden. Dieser Aufbau ermöglicht eine Werkzeugfreie Montage, die mit sehr geringen Kräften auskommt und dabei eine Baugruppe schafft, welche nicht mehr zerstörungsfrei demontiert werden kann.

Das Maulteil 1 ist also, wie dies vorstehend beschrieben ist, durch eine axiale Bewegung des Kulissenbauteils 220 relativ zu dem Nockenträgerbauteil 300 von einer Montagestellung in eine vollständig geöffnete Stellung bringbar. Die vollständig geöffnete Stellung ist dabei auch die Nullstellung des Clipapplikators.

Ein zweites Ausführungsbeispiel des ersten Aspekts der vorliegenden Erfindung ist unter Bezugnahme auf die Fig. 7 und 8 im Detail beschrieben.

Das Maulteil 1 entsprechend dem zweiten Ausführungsbeispiel hat im Wesentlichen denselben Aufbau wie das erste Ausführungsbeispiel, lediglich die Unterschiede zum ersten Ausführungsbeispiel sind hier erläutert.

Die Spange 340A, mit der das Nockenträgerbauteil 300 an der Schubstange des Rohrschaftinstruments anbringbar ist, ist so ausgestaltet, dass die beiden Arme nicht mehr zueinander hin elastisch verformbar sind, sondern dass diese parallel nach oben verformbar sin, um über entsprechende Gegenstücke an dem Betätigungsstab hinweg zu gleiten und mit diesen zu verrasten. Mit diesem Aufbau bedarf es beim anbau des Maulteils an einem Rohrschaft geringerer Montagekräfte. Gleichzeitig können mit diesem Aufbau größere Betätigungskräfte im Vergleich zu der Spange 340 des ersten Ausführungsbeispiels übertragen werden.

Darüber hinaus sind bei dem zweiten Ausführungsbeispiel die Hinterschneidungen zwischen Kulissenbahnen und Nocken stärker ausgebildet.

Sowohl die Kulissenbahnen als auch die Nocken dieses Ausführungsbeispiels weisen einen Querschnitt auf, der noch eher einer S-Form entspricht als dies bei dem ersten Ausführungsbeispiel der Fall ist.

Darüber hinaus ist in Fig. 8 gezeigt, dass die Krümmung des Bauchs 122b, 225b der Kulissenbahn 122, 225 kleiner als die Krümmung der Kehle 122k, 225k der Kulissenbahn 122, 225 ist und die Krümmung des Bauchs 302b, 305b der Nocke 302, 305 größer als die Krümmung der Kehle 302k, 305k der Nocke 302, 305 ist. Mehr noch ist die Krümmung des Bauchs 122b, 225b der Kulissenbahn 122, 225 größer als die Krümmung der Kehle 302k, 305k der Nocke 302, 305 ist und die Krümmung des Bauchs 302b, 305b der Nocke 302, 305 größer als die Krümmung der Kehle 122k, 225k der Kulissenbahn 122, 225 ist. Dies bedeutet, dass der Bauch immer stärker gekrümmt ist als die Kehle, in der er aufgenommen ist. Auf diese Weise bildet sich zwischen Bauch und Kehle im Querschnitt nur ein einziger Anlagepunkt aus und es kommt zu keiner Spreizwirkung des Bauchs in der Kehle. Bei mehr als einem Anlagepunkt im Querschnitt würde das aneinander entlang Gleiten von Kulissenelement und Nockenträgerbauteil gehemmt und die Reibung würde stark zunehmen. Außerdem könnte ein zu großer Bauch in einer zu kleinen Kehle dazu führen, dass sich Risse in der Kehle bilden, die das entsprechende Bauteil zerstören.

Eine weitere Besonderheit dieses Ausführungsbeispiels ist, dass der Kontaktpunkt zwischen Nocke 302, 305 und Kulissenbahn 122, 225 in einem Bereich ausgebildet ist, der gegenüber der Öffnungs-/Schließrichtung des Maulteils geneigt ist. Auf diese Weise wird die Kraft zwischen Kulissenelement 120, 220 und Nockenträgerbauteil 300 sicher im Bereich der Kulissenbahnen übertragen und es kommt zu einer unnötigen Erhöhung der Reibung zwischen diesen Bauteilen. Unnötige Reibung kann entstehen, wenn der Sandwichaufbau von Kulissenelementen 120, 220 und Nockenträgerbauteil 300 zusammen gedrückt wird. Dies wiederum kann leicht passieren, wenn die Kraftübertragung zu nahe an den Rändern der im Querschnitt gebogenen Kulissenbahnen und Nocken erfolgt.

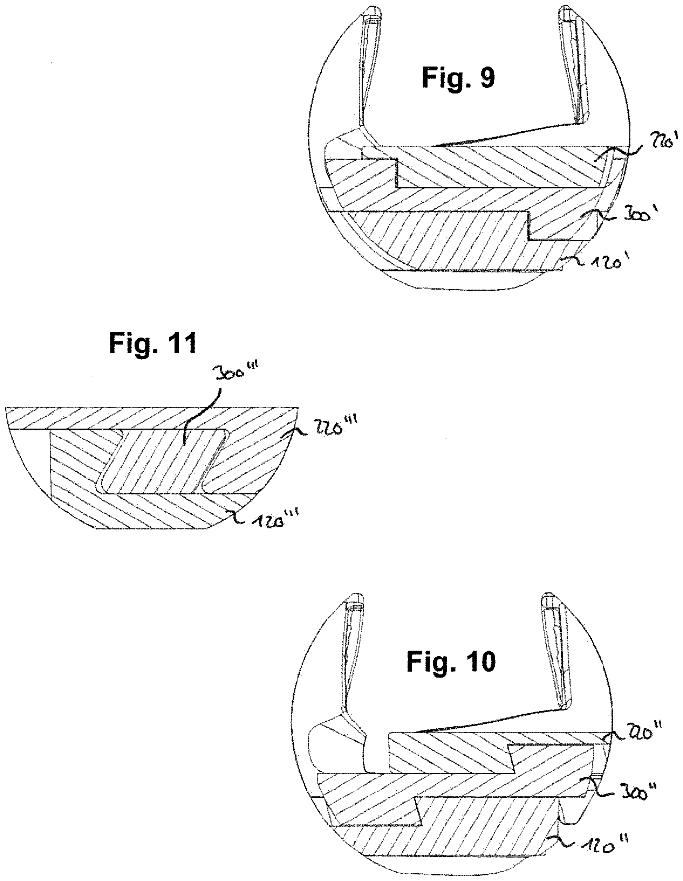

Ein drittes Ausführungsbeispiel des ersten Aspekts der vorliegenden Offenbarung ist unter Bezugnahme auf die Fig. 9 beschrieben.

Bei diesem Ausführungsbeispiel sind keine Hinterschneidungen zwischen den Kulissenelementen 120', 220' und dem Nockenträgerbauteil 300' ausgebildet. Vielmehr sind die Nocken 301 bis 306 und die Kulissenbahnen 121, 122, 123, 224, 225, 226 als vertikale Wände ausgebildet, die rechtwinklig von den Sandwichebenen des Maulteils 1 vorstehen. Die Sandwichebenen sind parallel zu der Ebene, in der der Öffnungs- bzw. Schließvorgang des Maulteil stattfindet. Mit einem solchen Aufbau muss mit anderen Mitteln verhindert werden, dass sich die Kulissenelemente 120', 220' von dem Nockenträgerbauteil 300' abheben. Der übrige Aufbau dieses Ausführungsbeispiels entspricht dem des ersten Ausführungsbeispiels.

Ein viertes Ausführungsbeispiel des ersten Aspekts der vorliegenden Erfindung ist unter Bezugnahme auf die Fig. 10 beschrieben.

Bei diesem Ausführungsbeispiel sind die Nocken 301 bis 306 und die Kulissenbahnen 121, 122, 123, 224, 225, 226 so ausgebildet, dass sie im Querschnitt eine Z-Form aufweisen. Auch mit einem solchen Aufbau ist ein Abheben der Kulissenelemente 120", 220" von dem Nockenträgerbauteil 300" sicher verhindert. Der übrige Aufbau entspricht dem des ersten Ausführungsbeispiels.

Ein fünftes Ausführungsbeispiel des ersten Aspekts der vorliegenden Erfindung ist unter Bezugnahme auf die Fig. 11 beschrieben.

Bei diesem Ausführungsbeispiel sind die Nocken 301 bis 306 des Nockenträgerbauteils 300'" zur Mittelachse hin verschoben, sodass das Nockenträgerbauteil auch seitlich zwischen den Kulissenelementen 120"', 220'" aufgenommen ist. Die Kulissenbahnen 121, 122, 123, 224, 225, 226 und die Nocken 301 bis 306 sind zudem so ausgebildet, dass sie im Querschnitt eine Z-Form aufweisen, die abgerundete Kanten aufweist. Man kann diese Form aber auch als S-Form bezeichnen, die einen geraden Mittelabschnitt aufweist. Auch mit einem solchen Aufbau ist ein Abheben der Kulissenelemente 120'", 220'" von dem Nockenträgerbauteil 300'" sicher verhindert. Der übrige Aufbau entspricht dem des ersten Ausführungsbeispiels.

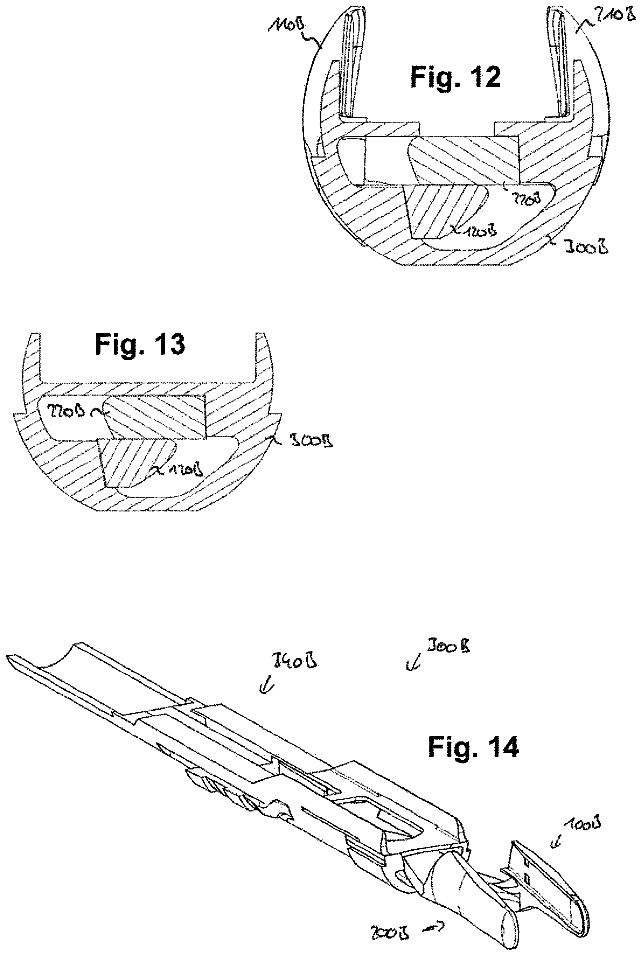

Ein sechstes Ausführungsbeispiel des ersten Aspekts der vorliegenden Erfindung ist unter Bezugnahme auf die Fig. 12 bis 14 im Detail beschrieben.

Im Gegensatz zu den vorstehenden Ausführungsbeispielen sind bei dem sechsten Ausführungsbeispiel die Branchen 100B, 200B bzw. deren Kulissenelemente 120B, 220B in dem Nockenträgerbauteil 300B aufgenommen. Man spricht in diesem Fall von einem außen liegenden Nockenträgerbauteil 300B. Bei diesem Ausführungsbeispiel werden die Branchen 100B, 200B an dem Nockenträgerbauteil 300B montiert, indem sie von distal in das Nockenträgerbauteil 300B eingeschoben werden. Ein Abheben der Kulissenelemente 120B, 220B von dem Nockenträgerbauteil 300B kann bei dieser Ausgestaltung nicht auftreten. Das Nockenträgerbauteil 300B kann so massiver ausgebildet werden, was aber zu Lasten der Kulissenelemente 120B, 220B geht.

Über die hier im Detail gezeigten Ausführungsbeispiele und Modifikationen ergeben sich für den Fachmann zahlreiche weitere vorteilhafte Kombinationen, die ebenfalls Gegenstand der vorliegenden Anmeldung sind.

## Patentansprüche

1. Maulteil für ein chirurgisches Rohrschaft-Instrument mit
einer ersten Branche (100) mit einem ersten Wirkbereich (110), und
einer zweiten Branche (200) mit einem zweiten Wirkbereich (210), wobei die erste Branche (100) und die zweite Branche (200) über je ein Kulissenelement (120, 220) verfügt und die Branchen (100, 200) in axialer Richtung gehalten sind, und
ein Nockenträgerbauteil (300) vorgesehen ist, welches relativ zu den Kulissenelemente (120, 220) in axialer Richtung verschiebbar ist, wobei
das Nockenträgerbauteil (300) wenigstens eine Nocke trägt und mindestens eines der Kulissenelemente (120, 220) wenigstens eine Kulissenbahn aufweist, wobei
die Kulissenelemente (120, 220) und das Nockenträgerbauteil (300) im Wesentlichen flächig ausgebildet sind, wobei
das Nockenträgerbauteil (300) und jedes Kulissenelement (120, 220) mindestens einen Bereich aufweisen, in dem sie eine Hinterschneidung ausbilden, sodass ein Abheben der Kulissenelemente (120, 220) von dem Nockenträgerbauteil (300) verhindert ist,
wobei das Maulteil **dadurch gekennzeichnet ist, dass** das Nockenträgerbauteil (300) zwischen den Kulissenelementen (120, 220) im Wesentlichen sandwichartig angeordnet ist.

2. Maulteil (1) für ein chirurgisches Rohrschaft-Instrument nach Anspruch 1,
**dadurch gekennzeichnet, dass**
mindestens ein Bereich der Hinterschneidung über den gesamten Bewegungsbereich des Kulissenelements (120, 220) zu dem Nockenträgerbauteil (300) von einer vollständig geöffneten Stellung zu einer vollständig geschlossenen Stellung des Maulteils vorhanden ist.

3. Maulteil für ein chirurgisches Rohrschaft-Instrument nach Anspruch 1,
**dadurch gekennzeichnet, dass**
die Nocke wenigstens über einen Teil der axialen Verschiebung des Nockenträgerbauteils (300) zu dem Kulissenelement (120, 220) an der Kulissenbahn anliegt, welche an dem Kulissenelement (120, 220) ausgebildet ist, wobei
die Nocke auf der der Kulissenbahn zugewandten Seite ihres axialen Querschnitts im Wesentlichen eine Z-Form, eine S-Form oder eine Kombination daraus aufweist.

4. Maulteil für ein chirurgisches Rohrschaft-Instrument nach Anspruch 1 oder 3,
**dadurch gekennzeichnet, dass**
die Nocke wenigstens über einen Teil der axialen Verschiebung des Nockenträgerbauteils (300) zu dem Kulissenelement (120, 220) an der Kulissenbahn anliegt, welche an dem Kulissenelement (120, 220) ausgebildet ist, wobei
die Kulissenbahn auf der der Nocke zugewandten Seite ihres axialen Querschnitts im Wesentlichen eine Z-Form, eine S-Form oder eine Kombination daraus aufweist.

5. Maulteil für ein chirurgisches Rohrschaft-Instrument nach einem der Ansprüche 3 oder 4,
**dadurch gekennzeichnet, dass**
Nocke und Kulissenbahn an den jeweils zugewandten Seiten ihres Querschnitts im Wesentlichen eine S-Form aufweisen und die Nocke und die Kulissenbahn auf diese Weise je einen Bauch und eine Kehle ausbilden und der Bauch eines Bauteils jeweils in die Kehle des anderen Bauteils vorsteht.

6. Maulteil für ein chirurgisches Rohrschaft-Instrument nach Anspruch 5,
**dadurch gekennzeichnet, dass**
zwischen Bauch und Kehle der Nocke und/oder der Kulissenbahn ein gerader Abschnitt ausgebildet ist, wobei dieser gerade Abschnitt gegenüber der Öffnungs-/Schließrichtung des Maulteils geneigt ist, weiter mehr als 7° und weniger als 20°.

7. Maulteil für ein chirurgisches Rohrschaft-Instrument nach einem der Ansprüche 5 oder 6,
**dadurch gekennzeichnet, dass**
die Krümmung des Bauchs der Kulissenbahn kleiner als die Krümmung der Kehle der Kulissenbahn ist und/oder die Krümmung des Bauchs der Nocke größer als die Krümmung der Kehle der Nocke ist.

8. Maulteil für ein chirurgisches Rohrschaft-Instrument nach einem der Ansprüche 5 bis 7,
**dadurch gekennzeichnet, dass**
die Krümmung des Bauchs der Kulissenbahn größer als die Krümmung der Kehle der Nocke ist und/oder die Krümmung des Bauchs der Nocke größer als die Krümmung der Kehle der Kulissenbahn ist.

9. Maulteil für ein chirurgisches Rohrschaft-Instrument nach einem der Ansprüche 1 bis 8,
**dadurch gekennzeichnet, dass**
die Richtung der Krafteinleitung von der Nocke in die Kulissenbahn gegenüber der Öffnungs-/Schließrichtung des Maulteils geneigt ist, in einem Bereich von bis zu 20°.

10. Maulteil für ein chirurgisches Rohrschaft-Instrument nach Anspruch 9,
**dadurch gekennzeichnet, dass**
die Oberfläche der Nocke im Bereich des Kontaktpunkts mit der Kulissenbahn der Mittelachse des Nockenträgerbauteils (300) zugewandt ist.

11. Maulteil für ein chirurgisches Rohrschaft-Instrument nach einem der Ansprüche 3 bis 10,
**dadurch gekennzeichnet, dass**
sich mindestens ein Kulissenelement (120, 220) im Wesentlichen über die gesamte Breite des Nockenträgerbauteils (300) erstreckt, sodass die jeweilige Nocke des Nockenträgerbauteils (300) im Wesentlichen durch das entsprechende Kulissenelement (120, 220) bedeckt ist.

12. Maulteil für ein chirurgisches Rohrschaft-Instrument nach Anspruch 1,
**dadurch gekennzeichnet, dass**
mindestens ein Kulissenelement (120, 220) wenigstens zwei Kulissenbahnen aufweist, welche mit einer entsprechenden Anzahl von Nocken an dem Nockenträgerbauteil (300) wenigstens über einen Teil der axialen Verschiebung der beiden Bauteile zueinander von einer vollständig geöffneten Stellung zu einer vollständig geschlossenen Stellung des Maulteils je eine Hinterschneidung ausbilden, wobei das Nockenträgerbauteil (300) und die Kulissenelemente (120, 220) durch die Hinterschneidungen ausschließlich zusammenbaubar sind, indem ineinander eindrehbar sind.

13. Maulteil für ein chirurgisches Rohrschaft-Instrument nach Anspruch 12,
**dadurch gekennzeichnet, dass**
sich das Kulissenelement und das Nockenträgerbauteil (300) nach einem eindrehenden Zusammenbau in einer Montagestellung befinden, die sich außerhalb der relativen axialen Verschiebung des Kulissenelements (120, 220) und des Nockenträgerbauteils (300) zueinander von der vollständig geöffneten Stellung zu der vollständig geschlossenen Stellung des Maulteils befindet.

14. Maulteil für ein chirurgisches Rohrschaft-Instrument mit
einer ersten Branche (100) mit einem ersten Wirkbereich (110), und
einer zweiten Branche (200) mit einem zweiten Wirkbereich (210), wobei
die erste Branche (100) und die zweite Branche (200) über je ein Kulissenelement (120, 220) verfügt und die Branchen (100, 200) in axialer Richtung gehalten sind, und
ein Nockenträgerbauteil (300) vorgesehen ist, welches relativ zu dem mindestens einem Kulissenelement (120, 220) in axialer Richtung verschiebbar ist, wobei
das Nockenträgerbauteil (300) wenigstens eine Nocke trägt und jedes Kulissenelement (120, 220) wenigstens eine Kulissenbahn aufweist, wobei
die Kulissenelemente (120, 220) im Wesentlichen flächig ausgebildet sind und im Wesentlichen schichtartig übereinander angeordnet sind,
**dadurch gekennzeichnet, dass** das Nockenträgerbauteil (300) im Wesentlichen eine hohlen Querschnitt aufweist, in dem die Kulissenelemente (120, 220) angeordnet sind.

15. Maulteil für ein chirurgisches Rohrschaft-Instrument nach Anspruch 14,
**dadurch gekennzeichnet, dass**
das Kulissenelement (120, 220) und das Nockenträgerbauteil (300) von einer Montagestellung durch eine axiale Bewegung relativ zueinander in eine vollständig geöffnete Stellung bringbar sind.

16. Maulteil für ein chirurgisches Rohrschaft-Instrument nach einem der vorstehenden Ansprüche 1 bis 15,
**dadurch gekennzeichnet, dass**
das Maulteil von der Schwenkachsenlosen Bauart ist.

17. Verfahren zum Zusammenbau einer Branche (100, 200) und eines Nockenträgerbauteils (300) für ein Maulteil nach einem der Ansprüche 1 bis 13, mit den folgenden Schritten:
Bereitstellen einer Branche (100, 200) und eines Nockenträgerbauteils (300) in einem Zustand, in dem ein Kulissenelement (120, 220) der Branche (100, 200) der zugehörigen Seite des Nockenträgerbauteils (300) zugewandt ist, wobei die Branche (100, 200) und das Nockenträgerbauteil (300) in zwei parallelen Ebenen angeordnet sind, die voneinander beabstandet sind, und die Längsachsen der beiden Bauteile einen bestimmten Winkel zwischen sich einnehmen,
Heranführen der Branche (100, 200) und des Nockenträgerbauteils (300) aneinander, bis diese in Kontakt gelangen, und
Drehen der Branche (100, 200) und des Nockenträgerbauteils (300) relativ zueinander in der durch die ihre Kontaktfläche bestimmten Ebene in der Weise, dass sich der Winkel zwischen ihren Längsachsen verringert, bis mindestens je eine Nocke des Nockenträgerbauteils (300) beiderseits des Kreuzungspunkts der beiden Längsachsen in Kontakt mit einer Kulissenbahn gelangt.

18. Verfahren zum Zusammenbau eines chirurgischen Rohrschaft-Instruments mit
einem Maulteil nach einem der Ansprüche 1 bis 13,
einem Schaftbauteil, und
einem Betätigungsstab,
mit den Schritten nach dem Anspruch 17 und den folgenden Schritten:
Anbringen einer anderen Branche (100, 200) an der gegenüberliegenden Seite des Nockenträgerbauteils (300), wobei dieser Schritt auch zu Begin des Verfahrens erfolgen kann, und
Einschieben des proximalen Endes des so erzeugten Maulteils in ein distales Ende des Schaftbauteils, wobei die Branche (100, 200) und/oder die andere Branche (100, 200) sowie das Nockenträgerbauteil (300) zu unterschiedlichen Zeitpunkten mit einem von dem Schaftbauteil oder dem Betätigungsstab in Eingriff gelangen, sodass dadurch eine axiale Verschiebung zwischen dem Nockenträgerbauteil (300) sowie der Branche (100, 200) und/oder der anderen Branche (100, 200) in eine Stellung erfolgt, die der vollständig geöffneten Stellung des Maulteils entspricht.

## Claims

1. Jaw member for a tubular shafted surgical instrument with
a first branch (100) having a first active region (110), and
a second branch (200) having a second active region (210),
wherein the first branch (100) and the second branch (200) have a respective link member (120, 220) and the branches (100, 200) are held in the axial direction, and
a cam support member (300) is provided which is slideable relative to the link members (120, 220) in the axial direction, wherein
the cam support member (300) carries at least one cam and at least one of the link members (120, 220) comprises at least one link path, wherein
the link members (120, 220) and the cam support member (300) are formed substantially planar, wherein
the cam support member (300) and each link member (120, 220) have at least one region in which they form an undercut so that lifting of the link member (120, 220) from the cam support member (300) is prevented,
wherein the jaw member is **characterized in that** the cam support member (300) is arranged substantially in a sandwich-like manner between the link members (120, 220).

2. Jaw member (1) for a tubular shafted surgical instrument according to claim 1,
**characterized in that**
at least one region of the undercut is present over the entire range of movement of the link member (120, 220) to the cam support member (300) from a fully open position to a fully closed position of the jaw member.

3. Jaw member for a tubular shafted surgical instrument according to claim 1,
**characterized in that**
the cam abuts at the link path, which is formed on the link element (120, 220), over at least a part of the axial displacement of the cam support member (300) to the link member (120, 220), wherein
the cam on the link path-facing side of their axial cross-section substantially has a Z-shape, an S-shape or a combination thereof.

4. Jaw member for a tubular shafted surgical instrument according to claim 1,
**characterized in that**
the cam abuts at the link path, which is formed on the link element (120, 220), over at least a part of the axial displacement of the cam support member (300) to the link member (120, 220), wherein
the link path on the cam-facing side of their axial cross-section substantially has a Z-shape, an S-shape or a combination thereof.

5. Jaw member for a tubular shafted surgical instrument according to one of claims 3 or 4,
**characterized in that**
cam and link path have at the respective facing sides of their cross-section substantially an S-shape and the cam and the link path in this way each form one belly and one groove and the belly of a component respectively protrudes in the groove of the other component.

6. Jaw member for a tubular shafted surgical instrument according to claim 5,
**characterized in that**
between belly and groove of the cam and/or of the link path, a straight portion is formed, wherein this straight portion is inclined relative to the opening/closing direction of the jaw member, furthermore more than 7° and less than 20°.

7. Jaw member for a tubular shafted surgical instrument according to one of claims 5 or 6,
**characterized in that**
the curvature of the belly of the link path is smaller than the curvature of the groove of the link path and/or the curvature of the belly of the cam is larger than the curvature of the groove of the cam.

8. Jaw member for a tubular shafted surgical instrument according to one of claims 5 to 7,
**characterized in that**
the curvature of the belly of the link path is larger than the curvature of the groove of the cam and/or the curvature of the belly of the cam is larger than the curvature of the groove of the link path.

9. Jaw member for a tubular shafted surgical instrument according to one of claims 1 to 8,
**characterized in that**
the direction of force application from the cam into the link path is inclined relative to the opening/closing direction of the jaw member, in a range of up to 20 °.

10. Jaw member for a tubular shafted surgical instrument according to claim 9,
**characterized in that**
the surface of the cam in the region of the contact point with the link path is facing the central axis of the cam support member (300).

11. Jaw member for a tubular shafted surgical instrument according to one of claims 3 to 10,
**characterized in that**
at least one link member (120, 220) substantially extends over the entire width of the cam support member (300), so that the respective cam of the cam support member (300) is substantially covered through the corresponding link member (120, 220).

12. Jaw member for a tubular shafted surgical instrument according to claim 1,
**characterized in that**
at least one link member (120, 220) comprises at least two link paths, which, with a corresponding number of cams on the cam support member (300) at least over a part of the axial displacement of the two components to each other from a fully open position to a fully closed position of the jaw member, form one respective undercut each, wherein the cam support member (300) and the link members (120, 220) can be exclusively assembled through the undercuts by screwing them into each other.

13. Jaw member for a tubular shafted surgical instrument according to claim 12,
**characterized in that**
the link member and the cam support member (300) are, after an inward-turning assembly, in a mounting position which is outside of the relative axial displacement of the link member (120, 220) and of the cam support member (300) to each other from the fully open position to the fully closed position of the jaw member.

14. Jaw member for a tubular shafted surgical instrument with
a first branch (100) having a first active region (110), and
a second branch (200) with a second active region (210),
wherein the first branch (100) and the second branch (200) each have a respective link member (120, 220) and the branches (100, 200) are held in the axial direction, and
a cam support member (300) is provided which is slideable relative to the at least one link member (120, 220) in the axial direction, wherein
the cam support member (300) carries at least one cam and each link member (120, 220) comprises at least one link path, wherein
the link members (120, 220) are formed substantially planar and are arranged substantially in a layer-like manner one above the other
**characterized in that**
the cam support member (300) has a substantially hollow cross section, in which the link members (120, 220) are arranged.

15. Jaw member for a tubular shafted surgical instrument according to claim 14,
**characterized in that**
the link member (120, 220) and the cam support member (300) can be brought from a mounting position to a fully open position by an axial movement relative to each other.

16. Jaw member for a tubular shafted surgical instrument according to one of the preceding claims 1 to 15,
**characterized in that**
the jaw member is of the pivot type without axis.

17. Method for assembling a branch (100, 200) and a cam support member (300) for a jaw member according to one of claims 1 to 13, comprising the steps of:
providing a branch (100, 200) and a cam support member (300) in a state in which a link member (120, 220) of the branch (100, 200) is facing the associated side of the cam support member (300), wherein said branch (100, 200) and the cam support member (300) are arranged in two parallel planes which are spaced from each other, and the longitudinal axes of the two components engage a certain angle between them,
advancing of the branch (100, 200) and the cam support member (300) towards each other until they are in contact, and
turning the branch (100, 200) and the cam support member (300) relative to each other in the plane specified by their contact surface in the manner that the angle between their longitudinal axes is reduced, until at least one respective cam of the cam support member (300) on either sides of the crossing point of the two longitudinal axes comes into contact with a link path.

18. Method for assembling of a surgical tubular shafted instrument with
a jaw member according to one of the claims 1 to 13,
a shaft member, and
an actuating rod,
comprising the steps of claim 17 and the steps of:
attaching a different branch (100, 200) on the opposite side of the cam support member (300), wherein this step can also be done at the beginning of the method, and
inserting the proximal end of the thus produced jaw member (1) into a distal end of the shaft member, wherein the branch (100, 200) and/or the other branch (100, 200) and the cam support member (300) come at different times into engagement with one of the shaft member or the actuating rod so that therefore an axial displacement between the cam support member (300) and the branch (100, 200) and/or the other branch (100, 200) in a position takes place which corresponds to the fully open position of the jaw member.

## Revendications

1. Elément de bec pour un instrument chirurgical à tige tubulaire avec
une première branche (100) avec une première zone active (110), et
une seconde branche (200) avec une seconde zone active (210),
dans lequel la première branche (100) et la seconde branche (200) disposent chacune d'un élément à coulisse (120, 220) et les branches (100, 200) sont maintenues dans la direction axiale, et
un composant de support de came (300) est prévu, lequel est mobile par rapport aux éléments à coulisse (120, 220) dans la direction axiale, dans lequel
le composant de support de came (300) porte au moins une came et au moins un des éléments à coulisse (120, 220) présente au moins une bande de coulissement, dans lequel
les éléments à coulisse (120, 220) et le composant de support de came (300) sont réalisés sensiblement plats, dans lequel
le composant de support de came (300) et chaque élément à coulisse (120, 220) présentent au moins une zone, dans laquelle ils réalisent une contre-dépouille de sorte qu'un soulèvement des éléments à coulisse (120, 220) soit empêché par le composant de support de came (300),
dans lequel l'élément de bec est **caractérisé en ce que** le composant de support de came (300) est agencé entre les éléments à coulisse (120, 220) sensiblement en sandwich.

2. Elément de bec (1) pour un instrument chirurgical à tige tubulaire selon la revendication 1,
**caractérisé en ce que**
au moins une zone de la contre-dépouille est présente au-dessus de la zone de mouvement entière de l'élément à coulisse (120, 220) vers le composant du support de came (300) d'une position complètement ouverte à une position complètement fermée de l'élément de bec.

3. Elément de bec pour un instrument chirurgical à tige tubulaire selon la revendication 1,
**caractérisé en ce que**
la came repose au moins sur une partie du coulissement axial du composant de support de came (300) vers l'élément à coulisse (120, 220) contre la bande de coulissement qui est réalisée au niveau de l'élément à coulisse (120, 220), dans lequel
la came présente sur le côté tourné vers la bande de coulissement de sa section transversale axiale sensiblement une forme de Z, une forme de S ou une combinaison de celles-ci.

4. Elément de bec pour un instrument chirurgical à tige tubulaire selon la revendication 1 ou 3,
**caractérisé en ce que**
la came repose au moins sur une partie du coulissement axial du composant de support de came (300) vers l'élément à coulisse (120, 220) contre la bande de coulissement qui est réalisée au niveau de l'élément à coulisse (120, 220), dans lequel
la bande de coulissement présente sur le côté tourné vers la came de sa section transversale axiale sensiblement une forme de Z, une forme de S ou une combinaison de celles-ci.

5. Elément de bec pour un instrument chirurgical à tige tubulaire selon l'une quelconque des revendications 3 ou 4,
**caractérisé en ce que**
la came et la bande de coulissement présentent au niveau des côtés tournés respectivement de leur section transversale sensiblement une forme de S et la came et la bande de coulissement réalisent de cette manière chacune un ventre et une gorge et le ventre d'un composant dépasse respectivement dans la gorge de l'autre composant.

6. Elément de bec pour un instrument chirurgical à tige tubulaire selon la revendication 5,
**caractérisé en ce que**
une section droite est réalisée entre le ventre et la gorge de la came et/ou de la bande de coulissement, dans lequel cette section droite est inclinée par rapport au sens d'ouverture/de fermeture de l'élément de bec, de plus de 7° et moins de 20°.

7. Elément de bec pour un instrument chirurgical à tige tubulaire selon l'une quelconque des revendications 5 ou 6,
**caractérisé en ce que**
la courbure du ventre de la bande de coulissement est inférieure à la courbure de la gorge de la bande de coulissement et/ou la courbure du ventre de la came est supérieure à la courbure de la gorge de la came.

8. Elément de bec pour un instrument chirurgical à tige tubulaire selon l'une quelconque des revendications 5 à 7,
**caractérisé en ce que**
la courbure du ventre de la bande de coulissement est supérieure à la courbure de la gorge de la came et/ou la courbure du ventre de la came est supérieure à la courbure de la gorge de la bande de coulissement.

9. Elément de bec pour un instrument chirurgical à tige tubulaire selon l'une quelconque des revendications 1 à 8,
**caractérisé en ce que**
la direction de l'introduction de force de la came dans la bande de coulissement est inclinée par rapport au sens d'ouverture/de fermeture de l'élément de bec, dans une plage de 20° maximum.

10. Elément de bec pour un instrument chirurgical à tige tubulaire selon la revendication 9,
**caractérisé en ce que**
la surface de la came dans la zone du point de contact avec la bande de coulissement est tournée vers l'axe médian du composant de support de came (300).

11. Elément de bec pour un instrument chirurgical à tige tubulaire selon l'une quelconque des revendications 3 à 10,
**caractérisé en ce que**
au moins un élément à coulisse (120, 220) s'étend sensiblement sur la largeur entière du composant de support de came (300) de sorte que la came respective du composant de support de came (300) soit recouverte sensiblement par l'élément à coulisse (120, 220) correspondant.

12. Elément de bec pour un instrument chirurgical à tige tubulaire selon la revendication 1,
**caractérisé en ce que**
au moins un élément à coulisse (120, 220) présente au moins deux bandes de coulissement qui réalisent avec un nombre correspondant de cames au niveau du composant de support de came (300) au moins sur une partie du coulissement axial des deux composants l'un par rapport à l'autre d'une position complètement ouverte à une position complètement fermée de l'élément de bec chacune une contre-dépouille, dans lequel le composant de support de came (300) et les éléments à coulisse (120, 220) peuvent être assemblés exclusivement par les contre-dépouilles, en étant vissés l'un dans l'autre.

13. Elément de bec pour un instrument chirurgical à tige tubulaire selon la revendication 12,
**caractérisé en ce que**
l'élément à coulisse et le composant de support de came (300) se trouvent après un assemblage de vissage dans une position de montage qui se trouve en dehors du coulissement axial relatif de l'élément à coulisse (120, 220) et du composant de support de came (300) l'un par rapport à l'autre de la position complètement ouverte à la position complètement fermée de l'élément de bec.

14. Elément de bec pour un instrument chirurgical à tige tubulaire avec
une première branche (100) avec une première zone active (110), et
une seconde branche (200) avec une seconde zone active (210),
dans lequel la première branche (100) et la seconde branche (200) disposent chacune d'un élément à coulisse (120, 220) et les branches (100, 200) sont maintenues dans la direction axiale, et
un composant de support de came (300) est prévu, lequel est mobile par rapport à l'au moins un élément à coulisse (120, 220) dans la direction axiale, dans lequel
le composant de support de came (300) porte au moins une came et chaque élément à coulisse (120, 220) présente au moins une bande de coulissement, dans lequel
les éléments à coulisse (120, 220) sont réalisés sensiblement plats et sont agencés sensiblement en couche l'un au-dessus de l'autre,
**caractérisé en ce que** le composant de support de came (300) présente sensiblement une section transversale creuse, dans laquelle les éléments à coulisse (120, 220) sont agencés.

15. Elément de bec pour un instrument chirurgical à tige tubulaire selon la revendication 14,
**caractérisé en ce que**
l'élément à coulisse (120, 220) et le composant de support de came (300) peuvent être amenés d'une position de montage par un mouvement axial l'un par rapport à l'autre dans une position complètement ouverte.

16. Elément de bec pour un instrument chirurgical à tige tubulaire selon l'une quelconque des revendications précédentes 1 à 15,
**caractérisé en ce que**
l'élément de bec est du type sans axe de pivotement.

17. Procédé d'assemblage d'une branche (100, 200) et d'un composant de support de came (300) pour un élément de bec selon l'une quelconque des revendications 1 à 13, avec les étapes suivantes :
la fourniture d'une branche (100, 200) et d'un composant de support de came (300) dans un état, dans lequel un élément à coulisse (120, 220) de la branche (100, 200) est tourné vers le côté afférent du composant de support de came (300), dans lequel la branche (100, 200) et le composant de support de came (300) sont agencés dans deux plans parallèles qui sont espacés l'un de l'autre, et les axes longitudinaux des deux composants occupent un angle déterminé entre eux,
l'approche de la branche (100, 200) et du composant de support de came (300) l'un de l'autre jusqu'à ce que ceux-ci parviennent en contact, et
la rotation de la branche (100, 200) et du composant de support de came (300) l'un par rapport à l'autre dans le plan déterminé par la leur surface de contact de telle manière que l'angle entre leurs axes longitudinaux diminue jusqu'à ce qu'au moins une came du composant de support de came (300) de part et d'autre du point d'intersection des deux axes longitudinaux parvienne chacune en contact avec une bande de coulissement.

18. Procédé d'assemblage d'un instrument chirurgical à tige tubulaire avec
un élément de bec selon l'une quelconque des revendications 1 à 13,
un composant de tige, et
une barre d'actionnement,
avec les étapes selon la revendication 17 et les étapes suivantes :
le montage d'une autre branche (100, 200) au niveau du côté opposé du composant de support de came (300), dans lequel cette étape peut aussi être effectuée au début du procédé et
l'enfoncement de l'extrémité proximale de l'élément de bec ainsi généré dans une extrémité distale du composant de tige, dans lequel la branche (100, 200) et/ou l'autre branche (100, 200) ainsi que le composant de support de came (300) parviennent en prise à des moments différents avec un du composant de tige ou de la barre d'actionnement de sorte qu'ainsi un coulissement axial entre le composant de support de came (300) ainsi que la branche (100, 200) et/ou l'autre branche (100, 200) soit effectué dans une position qui correspond à la position complètement ouverte de l'élément de bec.
